(19) Europäisches Patentamt / European Patent Office / Office européen des brevets

(11) **EP 4 722 341 A1**

(12) **EUROPEAN PATENT APPLICATION**
published in accordance with Art. 153(4) EPC

(43) Date of publication:
**08.04.2026 Bulletin 2026/15**

(21) Application number: **24815578.0**

(22) Date of filing: **30.05.2024**

(51) International Patent Classification (IPC):
*C12N 5/07* (2010.01)   *C12N 5/02* (2006.01)
*C12N 5/10* (2006.01)   *C12N 5/078* (2010.01)
*C12N 5/0786* (2010.01)   *C12N 5/0787* (2010.01)
*C12N 5/0789* (2010.01)

(52) Cooperative Patent Classification (CPC):
**C12N 5/0006; C12N 5/06; C12N 5/10**

(86) International application number:
**PCT/JP2024/019905**

(87) International publication number:
**WO 2024/248098 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.06.2023 JP 2023091457**
**27.07.2023 JP 2023122303**

(71) Applicants:
• **Sekisui Chemical Co., Ltd.**
**Osaka-shi, Osaka 530-8565 (JP)**
• **CiRA Foundation**
**Kyoto-shi, Kyoto 606-8397 (JP)**

(72) Inventors:
• **NAKAMURA, Yuuta**
**Mishima-gun, Osaka 618-0021 (JP)**
• **YANAGISAWA, Teruhiko**
**Tsukuba-shi, Ibaraki 300-4292 (JP)**
• **SANGHAVI, Rutvi Ravin**
**Mishima-gun, Osaka 618-0021 (JP)**
• **SHIMASAKI, Noriko**
**Kyoto-shi, Kyoto 606-8397 (JP)**
• **SHIMIZU, Eiko**
**Kyoto-shi, Kyoto 606-8397 (JP)**

(74) Representative: **Ter Meer Steinmeister & Partner Patentanwälte mbB**
**Nymphenburger Straße 4**
**80335 München (DE)**

Remarks:
The complete document including Reference Table(s) and the Sequence Listing(s) can be downloaded from the EPO website

(54) **METHOD FOR PRODUCING HEMATOPOIETIC PROGENITOR CELL AND METHOD FOR PRODUCING LEUKOCYTE**

(57)    Provided is a method for producing hematopoietic progenitor cells by which hematopoietic progenitor cells can be stably obtained from pluripotent stem cells, and the efficiency of inducing differentiation from pluripotent stem cells into hematopoietic progenitor cells can be enhanced. A method for producing hematopoietic progenitor cells according to the present invention includes: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing hematopoietic progenitor cells.

EP 4 722 341 A1

[FIG. 1]

(a)

(b)

## Description

### TECHNICAL FIELD

The present invention relates to a method for producing hematopoietic progenitor cells. The present invention also relates to a method for producing leukocytes.

### BACKGROUND ART

In recent years, attempts have been made to induce differentiation of hematopoietic progenitor cells (hematopoietic stem cells) from induced pluripotent stem cells (iPS cells) and to induce differentiation of leukocytes. The iPS cells usually differentiate into hematopoietic progenitor cells through mesoderm and hemogenic endothelial cells. Hematopoietic progenitor cells also differentiate into leukocytes.

For example, Non-Patent Document 1 below describes that monocytes, which are one type of leukocytes, are induced to differentiate from iPS cells using a plate coated with laminin on the entire bottom surface.

Meanwhile, Patent Document 1 below discloses a method for inducing differentiation of ectodermal cells from pluripotent stem cells using a cell culture substrate having an island-shaped region (A) having cell adhesiveness and cell proliferation and having an area of 0.001 to 5 mm$^2$ and a region (B), which is adjacent to the region (A), having no adhesiveness or cell proliferation. However, the method described in Patent Document 1 is a method for inducing differentiation of pluripotent stem cells into ectodermal cells, and Patent Document 1 does not describe differentiation of pluripotent stem cells into mesoderm, and thus does not describe differentiation of pluripotent stem cells into hematopoietic progenitor cells.

Patent Document 2 described below describes a scaffold for cell culture including a substrate and protrusions configured by being patterned in a dot shape or a line shape on the substrate. The protrusion contains a synthetic resin, and the synthetic resin contains a polyvinyl alcohol derivative or a poly(meth)acrylic resin. In Examples of Patent Document 2, it is described that iPS cells are cultured using a container for cell culture including the scaffold for cell culture. However, Patent Document 2 does not describe differentiation of iPS cells.

#### Related Art Document

#### Patent Documents

Patent Document 1: JP 2021-158960 A
Patent Document 2: WO 2021/024943 A1

#### Non-Patent Document

Non-Patent Document 1: High-Yield Human Induced Pluripotent Stem Cell-Derived Monocytes and Macrophages Are Functionally Comparable With Primary Cells (Front Cell Dev Biol. 2021 Apr 13; 9:656867. doi: 10.3389/fcell.2021.656867)

### SUMMARY OF THE INVENTION

### PROBLEMS TO BE SOLVED BY THE INVENTION

As described in Non-Patent Document 1, conventionally, an attempt has been made to differentiate iPS cells into blood cells such as hematopoietic progenitor cells and leukocytes using a plate coated with laminin on the entire bottom surface. However, in the conventional method, the number of differentiated cells (hematopoietic progenitor cells or leukocytes) to be obtained varies or the expression ratio of the differentiation marker varies depending on the seeding conditions of the pluripotent stem cells and the type and state of the pluripotent stem cells, and thus it is difficult to stably obtain the differentiated cells.

An object of the present invention is to provide a method for producing hematopoietic progenitor cells by which hematopoietic progenitor cells can be stably obtained from pluripotent stem cells, and the efficiency of inducing differentiation from pluripotent stem cells into hematopoietic progenitor cells can be enhanced. An object of the present invention is to provide a method for producing leukocytes by which leukocytes can be stably obtained from pluripotent stem cells, and the efficiency of inducing differentiation from pluripotent stem cells into leukocytes can be enhanced.

## MEANS FOR SOLVING THE PROBLEMS

In the present specification, the following method for producing hematopoietic progenitor cells and method for producing leukocytes are disclosed.

Item 1. A method for producing hematopoietic progenitor cells, the method including: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing hematopoietic progenitor cells.

Item 2. The method for producing hematopoietic progenitor cells according to item 1, in which an average value of equivalent circle diameters of an upper surface of the cell scaffold is 200 $\mu$m or more and 3000 $\mu$m or less.

Item 3. The method for producing hematopoietic progenitor cells according to item 1 or 2, in which an average value of distances between centers of gravity of an upper surface of the cell scaffold is 0.5 mm or more and 10 mm or less.

Item 4. The method for producing hematopoietic progenitor cells according to any one of items 1 to 3, in which the cell scaffold contains a peptide-conjugated resin having a synthetic resin moiety and a peptide moiety.

Item 5. The method for producing hematopoietic progenitor cells according to item 4, in which the peptide-conjugated resin has a polyvinyl acetal resin moiety and a peptide moiety.

Item 6. The method for producing hematopoietic progenitor cells according to any one of items 1 to 5, in which a region where the cell scaffold is not disposed on the first surface of the substrate has a region subjected to a cell low adhesion treatment.

Item 7. The method for producing hematopoietic progenitor cells according to any one of items 1 to 6, in which the pluripotent stem cells are iPS cells.

Item 8. A method for producing leukocytes, the method including a step of inducing differentiation of a cell population containing hematopoietic progenitor cells obtained by the method for producing hematopoietic progenitor cells according to any one of items 1 to 7 into a cell population containing monocytes, lymphocytes, or granulocytes.

Item 9. A method for producing leukocytes, the method including: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing monocytes, lymphocytes, or granulocytes.

Item 10. The method for producing leukocytes according to item 9, in which an average value of equivalent circle diameters of an upper surface of the cell scaffold is 200 $\mu$m or more and 3000 $\mu$m or less.

Item 11. The method for producing leukocytes according to item 9 or 10, in which an average value of distances between centers of gravity of an upper surface of the cell scaffold is 0.5 mm or more and 10 mm or less.

Item 12. The method for producing leukocytes according to any one of items 9 to 11, in which the cell scaffold contains a peptide-conjugated resin having a synthetic resin moiety and a peptide moiety.

Item 13. The method for producing leukocytes according to item 12, in which the peptide-conjugated resin has a polyvinyl acetal resin moiety and a peptide moiety.

Item 14. The method for producing leukocytes according to any one of items 9 to 13, in which a region where the cell scaffold is not disposed on the first surface of the substrate has a region subjected to a cell low adhesion treatment.

Item 15. The method for producing leukocytes according to any one of items 9 to 14, in which the pluripotent stem cells are iPS cells.

## EFFECT OF THE INVENTION

A method for producing hematopoietic progenitor cells according to the present invention includes: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing hematopoietic progenitor cells. Since the method for producing hematopoietic progenitor cells according to the present invention is provided with the above-described configuration, hematopoietic progenitor cells can be stably obtained from pluripotent stem cells, and the efficiency of inducing differentiation from pluripotent stem cells into hematopoietic progenitor cells can be enhanced.

A method for producing leukocytes according to the present invention includes: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing monocytes, lymphocytes, or granulocytes. Since the method for producing leukocytes according to the present invention is provided with the above-described configuration, leukocytes can be stably obtained from pluripotent stem cells, and the efficiency of inducing differentiation from pluripotent stem cells into leukocytes can be enhanced.

**BRIEF DESCRIPTION OF DRAWINGS**

[Fig. 1] Figs. 1(a) and 1(b) are a plan view and a cross-sectional view schematically showing an example of a cell culture substrate usable in a method for producing hematopoietic progenitor cells and a method for producing leukocytes.
[Fig. 2] Fig. 2 is an example of a flow of inducing differentiation of leukocytes (monocytes, lymphocytes, or granulocytes) from iPS cells via hematopoietic progenitor cells.

**MODES FOR CARRYING OUT THE INVENTION**

Hereinafter, details of the present invention will be described.
A method for producing hematopoietic progenitor cells according to the present invention includes: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing hematopoietic progenitor cells.
Since the method for producing hematopoietic progenitor cells according to the present invention is provided with the above-described configuration, hematopoietic progenitor cells can be stably obtained from pluripotent stem cells, and the efficiency of inducing differentiation from pluripotent stem cells into hematopoietic progenitor cells can be enhanced.
The present inventors have found that hematopoietic progenitor cells can be stably obtained from pluripotent stem cells and the efficiency of inducing differentiation from pluripotent stem cells into hematopoietic progenitor cells can be enhanced by controlling the size of the cell cluster using the cell culture substrate. In the method for producing hematopoietic progenitor cells according to the present invention, hematopoietic progenitor cells can be stably obtained from pluripotent stem cells as compared with the case of using a conventional cell culture substrate in which the entire bottom surface is coated with a cell scaffold. For example, in the method for producing hematopoietic progenitor cells according to the present invention, hematopoietic progenitor cells can be stably obtained even under conditions where the seeding density of pluripotent stem cells is high, as compared with the case of using a conventional cell culture substrate in which the entire bottom surface is coated with a cell scaffold.
A method for producing leukocytes according to the present invention includes: a cell cluster forming step of culturing pluripotent stem cells using a cell culture substrate including a substrate and a cell scaffold disposed in a dot pattern on a first surface of the substrate and forming a cell cluster on a surface of the cell scaffold; and a differentiation induction step of inducing differentiation of the obtained cell cluster into a cell population containing monocytes, lymphocytes, or granulocytes.
Since the method for producing leukocytes according to the present invention is provided with the above-described configuration, leukocytes can be stably obtained from pluripotent stem cells, and the efficiency of inducing differentiation from pluripotent stem cells into leukocytes can be enhanced.
The present inventors have found that leukocytes can be stably obtained from pluripotent stem cells and the efficiency of inducing differentiation from pluripotent stem cells into leukocytes can be enhanced by controlling the size of the cell cluster using the cell culture substrate. In the method for producing leukocytes according to the present invention, leukocytes can be stably obtained from pluripotent stem cells, and the efficiency of inducing differentiation from pluripotent stem cells into leukocytes can be enhanced, as compared with the case of using a conventional cell culture substrate in which the entire bottom surface is coated with a cell scaffold. In the method for producing leukocytes according to the present invention, the number of leukocytes obtained from one pluripotent stem cell can be increased as compared with the case of using a conventional cell culture substrate in which the entire bottom surface is coated with a cell scaffold.
In the method for producing hematopoietic progenitor cells and the method for producing leukocytes according to the present invention, the following possibility is considered as the reason for exerting the above effect. In a conventional method using a cell culture substrate in which the entire bottom surface is coated with a cell scaffold, the size of a cell cluster and the distance between cell clusters are usually controlled by reducing the seeding density of pluripotent stem cells so that cell clusters to be formed do not adhere to each other. However, in this method, since the arrangement of the cell clusters is random, some or many cell clusters adhere to each other, and it is difficult to control the size of the cell cluster and the distance between the cell clusters. The present inventors have found that the reason why differentiated cells (hematopoietic progenitor cells or leukocytes) cannot be stably obtained or the efficiency of inducing differentiation cannot be enhanced in the conventional method is that the size of the cell cluster and the distance between the cell clusters greatly vary. On the other hand, in the production method according to the present invention, since the cell culture substrate having a cell scaffold disposed in a dot pattern is used, the size of the cell cluster, the number of cell clusters, and the distance between the cell clusters can be controlled. Therefore, the differentiation-inducing factor is likely to uniformly spread in the cells. Cytokines secreted from the cell cluster become uniform, and promotion of differentiation induction between cell clusters becomes uniform (paracrine effect). Thereby, differentiated cells (hematopoietic progenitor cells or leukocytes) can be stably obtained from pluripotent stem cells, and the efficiency of inducing differentiation from pluripotent stem cells into differentiated cells (hematopoietic progenitor cells or leukocytes) can be enhanced. In the production method according to the present invention, for example, it is possible to effectively suppress variations in the number of differentiated cells (hematopoietic progenitor cells or leukocytes) to be obtained and the expression ratio of the differentiation marker, and to stably obtain differentiated cells. The number of differentiated cells (hematopoietic progenitor cells or leukocytes) obtained from one pluripotent stem cell can be increased, and the efficiency of inducing differentiation can be enhanced. However, the above is presumptive, and the reason why the above effect can be exhibited in the production method according to the present invention is not limited thereto.

First, a cell culture substrate usable in the method for producing hematopoietic progenitor cells and the method for producing leukocytes according to the present invention will be described.

[Cell culture substrate]

The cell culture substrate includes a substrate and a cell scaffold disposed in a dot pattern on a first surface of the substrate. The first surface of the substrate has a region where the cell scaffold is disposed and a region where the cell scaffold is not disposed.

Hereinafter, an example of the cell culture substrate will be described with reference to the drawings.

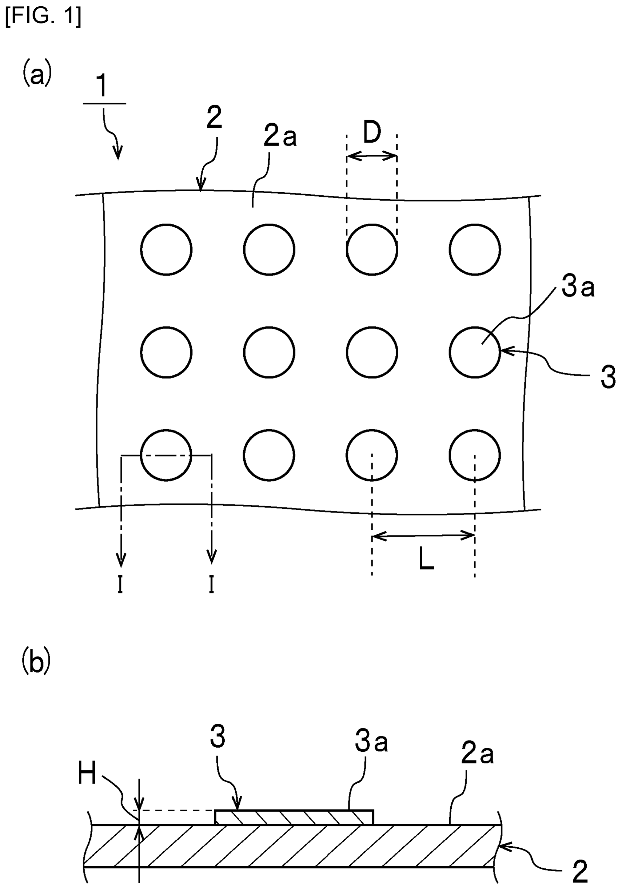

Figs. 1(a) and 1(b) are a plan view and a cross-sectional view schematically showing an example of a cell culture substrate usable in a method for producing hematopoietic progenitor cells and a method for producing leukocytes. Fig. 1(a) is a plan view, and Fig. 1(b) is a cross-sectional view taken along line I-I of Fig. 1(a).

A cell culture substrate 1 illustrated in Fig. 1 includes a substrate 2 and a cell scaffold 3. The cell scaffold 3 is disposed in a dot pattern on a first surface 2a of the substrate 2. The first surface 2a of the substrate 2 and the cell scaffold 3 are in direct contact with each other. The cell scaffold 3 is periodically disposed on the first surface 2a of the substrate 2, and is disposed at equal intervals. The cell scaffold 3 has an upper surface 3a. The upper surface 3a of the cell scaffold 3 is a surface opposite to the first surface 2a side of the substrate 2 in the cell scaffold 3.

In the cell culture substrate 1, the outer shape of the upper surface 3a of the cell scaffold 3 is circular. Reference sign D in Fig. 1(a) is a diameter of the upper surface 3a of the cell scaffold 3. In the cell culture substrate 1, since the outer shape of the upper surface 3a of the cell scaffold 3 is circular, the diameter D of the upper surface 3a of the cell scaffold 3 corresponds to the equivalent circle diameter of the upper surface 3a of the cell scaffold 3. Reference sign L in Fig. 1(a) is a distance between centers of gravity of the upper surface 3a of the cell scaffold 3. In the cell culture substrate 1, since the outer shape of the upper surface 3a of the cell scaffold 3 is circular, the distance L between centers of gravity of the upper surface 3a of the cell scaffold 3 is a distance between centers of a circle that is the outer shape of the upper surface 3a of the cell scaffold 3. In Fig. 1(b), reference sign H represents the height of the cell scaffold 3. The height H of the cell scaffold 3 is a distance from the first surface 2a of the substrate 2 to the upper surface 3a of the cell scaffold 3.

(Substrate)

The cell culture substrate includes a substrate (cell culture substrate body). The material, size, and shape of the substrate are not particularly limited. As the substrate, a conventionally known substrate can be used.

Examples of the material of the substrate include synthetic resins, metals, and glass. Examples of the synthetic resin include polystyrene, polyethylene, polypropylene, polyethersulfone, polycarbonate, polyester, polyisoprene, a cycloolefin polymer, polyimide, polyamide, polyamideimide, a (meth)acrylic resin, an epoxy resin, and silicone.

Examples of the shape of the substrate include a plate shape, a film shape, a porous membrane, and a bag shape. The substrate may be a container.

The substrate is preferably a container. The cell culture substrate is preferably a cell culture container. The cell culture substrate is preferably a cell culture container including a container (cell culture container body) and a cell scaffold disposed in a dot pattern on a first surface of the container. The shape and size of the container (cell culture container body) are not particularly limited. Examples of the container (cell culture container body) include a 2 to 384-well plate, a single-layer flask, a multi-layer flask, a multi-plane flask, a dish, a roller bottle, a bag, an insert cup, and a microchannel chip. The first surface of the substrate is preferably a bottom surface of the container.

(Cell scaffold)

The cell culture substrate includes a cell scaffold. The cell scaffold is disposed in a dot pattern on the first surface of the substrate. The cell scaffold has an upper surface. The upper surface of the cell scaffold is a surface opposite to the first surface side of the substrate in the cell scaffold.

The outer shape of the upper surface of the cell scaffold is not particularly limited. The outer shape of the upper surface of the cell scaffold may be a circle, an ellipse, a polygon, or a shape other than these shapes.

The number (number of dots) of the cell scaffolds disposed on the first surface of the substrate can be appropriately changed according to the type, shape, size, and the like of the substrate. The number (number of dots) of the cell scaffolds disposed on the first surface of the substrate may be, for example, 1 or more, 3 or more, 5 or more, or 7 or more, and may be 150 or less, 120 or less, 90 or less, or 60 or less, per 1 cm$^2$ of the area of the first surface of the substrate.

The cell scaffold may be arranged at equal intervals or may not be arranged at equal intervals on the first surface of the substrate. The cell culture substrate may have a region in which the cell scaffold is arranged at equal intervals and a region in which the cell scaffold is not arranged at equal intervals on the first surface of the substrate. The cell scaffold may or may not be periodically disposed on the first surface of the substrate. The cell culture substrate may have a region in which the cell scaffold is periodically arranged and a region in which the cell scaffold is not periodically arranged on the first surface of the substrate.

The average value of aspect ratios of the upper surface of the cell scaffold is preferably 6 or less, more preferably 5 or less, still more preferably 4 or less, further preferably 3 or less, even further preferably 2 or less, particularly preferably 1.5 or less, and most preferably 1.25 or less. When the average value of aspect ratios is the upper limit or less, the cell cluster can be more favorably formed. Note that the average value of aspect ratios of the upper surface of the cell scaffold may be 1 or more or more than 1.

The aspect ratio is a ratio (major axis/minor axis) of a major axis of the upper surface of the cell scaffold to a minor axis of the upper surface of the cell scaffold. The aspect ratio can be calculated, for example, by photographing the surface of the cell culture substrate with a microscope and analyzing the micrograph using commercially available image analysis software. The average value of aspect ratios is determined by averaging the aspect ratios of the upper surface of a plurality of cell scaffolds. The average value of aspect ratios is preferably determined by averaging the aspect ratios of the upper surface of 10 or more cell scaffolds arbitrarily selected from the entire first surface of the substrate. When there are less than 10 cell scaffolds on the first surface of the substrate, it is preferable to determine the average value of aspect ratios by averaging the aspect ratios of the upper surfaces of all the cell scaffolds.

The average value of equivalent circle diameters of the upper surface of the cell scaffold is preferably 200 $\mu$m or more, more preferably 300 $\mu$m or more, still more preferably 350 $\mu$m or more, further preferably 400 $\mu$m or more, and particularly preferably 500 $\mu$m or more, and is preferably 3000 $\mu$m or less, more preferably 2500 $\mu$m or less, still more preferably 2000 $\mu$m or less, further preferably 1500 $\mu$m or less, and particularly preferably 1000 $\mu$m or less. When the average value of the equivalent circle diameters is the lower limit or more and the upper limit or less, the size of the cell cluster can be easily controlled in a suitable range, and the effect of the present invention can be more effectively exhibited.

The equivalent circle diameter means a diameter of a circle having the same area as the area of the upper surface of the cell scaffold. The equivalent circle diameter can be calculated by the following equation. The equivalent circle diameter can be calculated, for example, by photographing the surface of the cell culture substrate with a microscope and analyzing the micrograph using commercially available image analysis software. The average value of equivalent circle diameters is determined by averaging the equivalent circle diameters of the upper surface of a plurality of cell scaffolds. The average value of equivalent circle diameters is preferably determined by averaging the equivalent circle diameters of the upper surface of 10 or more cell scaffolds arbitrarily selected from the entire first surface of the substrate. When there are less than 10 cell scaffolds on the first surface of the substrate, it is preferable to determine the average value of equivalent circle diameters by averaging the equivalent circle diameters of the upper surfaces of all the cell scaffolds.

[Mathematical 1]

$$D = 2 \times \sqrt{S} \div \sqrt{\pi}$$

D: Equivalent circle diameter of upper surface of cell scaffold
S: Area of upper surface of cell scaffold

The average value of the distances between centers of gravity of the upper surface of the cell scaffold is preferably 0.5 mm or more, more preferably 0.7 mm or more, still more preferably 1 mm or more, further preferably 1.5 mm or more, and particularly preferably 2 mm or more, and is preferably 10 mm or less, more preferably 9 mm or less, still more preferably 8 mm or less, further preferably 7 mm or less, even further preferably 6 mm or less, and particularly preferably 5 mm or less. When the average value of the distances between centers of gravity is the lower limit or more and the upper limit or less, the distance between the cell clusters can be easily controlled in a suitable range, and the effect of the present invention can be more effectively exhibited.

The distance between centers of gravity is a distance from the center of gravity of the upper surface of the cell scaffold to the center of gravity of the upper surface of the cell scaffold closest to the center of gravity of the upper surface of the cell scaffold (distance between one center of gravity and another center of gravity closest to the one center of gravity). The distance between centers of gravity can be calculated, for example, by photographing the surface of the cell culture substrate with a microscope and analyzing the micrograph using commercially available image analysis software. The average value of distances between centers of gravity is determined by averaging the distances between centers of gravity of the upper surface of a plurality of cell scaffolds. The average value of distances between centers of gravity is preferably determined by averaging the distances between centers of gravity of the upper surface of 10 or more cell scaffolds arbitrarily selected from the entire first surface of the substrate. When there are less than 10 cell scaffolds on the first surface of the substrate, it is preferable to determine the average value of distances between centers of gravity by averaging the distances between centers of gravity of the upper surfaces of all the cell scaffolds.

The average value of heights of the cell scaffold may be 1 nm or more, 5 nm or more, 10 nm or more, 15 nm or more, or 20 nm or more, and may be 10 $\mu$m or less, 5 $\mu$m or less, 2 $\mu$m or less, or 1 $\mu$m or less. When the average value of heights of the cell scaffold is the lower limit or more, the adhesion of cells to the cell scaffold can be enhanced, and the proliferation of cells can be enhanced. When the average value of heights of the cell scaffold is the upper limit or less, the production cost of the cell culture substrate can be reduced.

The height of the cell scaffold is a distance from the first surface of the substrate to an upper surface of the cell scaffold. The height of the cell scaffold is preferably a distance from the first surface of the substrate to the center of gravity of the upper surface of the cell scaffold. The average value of heights is determined by averaging the heights of a plurality of cell scaffolds. The average value of heights is preferably determined by averaging the heights of 10 or more cell scaffolds arbitrarily selected from the entire first surface of the substrate. When there are less than 10 cell scaffolds on the first surface of the substrate, it is preferable to determine the average value of heights by averaging the heights of all the cell scaffolds.

Note that examples of the microscope used for measuring the aspect ratio, equivalent circle diameter, and the distance between centers of gravity of the upper surface of the cell scaffold, and the height of the cell scaffold include "BZ-X800" manufactured by KEYENCE CORPORATION, and examples of the application for analysis include "BZ-H4M/measurement application" manufactured by KEYENCE CORPORATION.

Examples of the component contained in the cell scaffold include a cell adhesive protein and a synthetic resin. Only one kind of the component may be used, or two or more kinds thereof may be used in combination.

Examples of the cell adhesive protein include laminin, vitronectin, and collagen. The component contained in the cell scaffold may be a component containing a cell adhesive protein such as Matrigel. Examples of the Matrigel include Matrigel derived from mouse sarcoma.

As described later, the synthetic resin may include a synthetic resin having a synthetic resin moiety and a peptide moiety (synthetic resin to which a peptide is bonded). The synthetic resin may include a synthetic resin having no peptide moiety (synthetic resin to which no peptide is bonded). Examples of the synthetic resin include a polyvinyl alcohol derivative, a peptide-conjugated polyvinyl alcohol derivative having a polyvinyl alcohol derivative moiety and a peptide moiety, a (meth)acrylic copolymer, and a peptide-conjugated (meth)acrylic copolymer having a poly(meth)acrylic acid ester moiety and a peptide moiety. Examples of the polyvinyl alcohol derivative include a polyvinyl acetal resin.

Hereinafter, in the present specification, the "synthetic resin having a synthetic resin moiety and a peptide moiety" may be referred to as "peptide-conjugated resin".

The cell scaffold preferably contains a cell adhesive protein or a synthetic resin, more preferably contains laminin, vitronectin, or a peptide-conjugated resin, and still more preferably contains a peptide-conjugated resin. In this case, the effects of the present invention can be more effectively exhibited.

<Peptide-conjugated resin>

From the viewpoint of more effectively exhibiting the effects of the present invention and the viewpoint of enhancing the production efficiency of the cell culture substrate, the cell scaffold preferably contains a peptide-conjugated resin having a synthetic resin moiety and a peptide moiety. The peptide-conjugated resin is a synthetic resin to which a peptide is bonded. The peptide-conjugated resin has a synthetic resin moiety and a peptide moiety. Only one kind of the peptide-conjugated resin may be used, or two or more kinds thereof may be used in combination.

The synthetic resin moiety preferably has a polyvinyl alcohol derivative moiety or a poly(meth) acrylic acid ester moiety, and is more preferably a polyvinyl alcohol derivative moiety or a poly(meth)acrylic acid ester moiety. The peptide-conjugated resin preferably has a polyvinyl alcohol derivative moiety or a poly(meth)acrylic acid ester moiety and a peptide moiety. In this case, the effects of the present invention can be more effectively exhibited. The peptide-conjugated resin may have both a polyvinyl alcohol derivative moiety and a poly(meth) acrylic acid ester moiety.

In the peptide-conjugated resin having a polyvinyl alcohol derivative moiety, it is preferable that the polyvinyl alcohol derivative moiety and the peptide moiety are bonded to each other via a linker moiety. Therefore, the peptide-conjugated resin having a polyvinyl alcohol derivative moiety preferably has a polyvinyl alcohol derivative moiety, a peptide moiety, and a linker moiety.

In the peptide-conjugated resin having a poly(meth)acrylic acid ester moiety, the poly(meth)acrylic acid ester moiety and the peptide moiety may be bonded to each other via a linker moiety, or may be bonded directly to each other without a linker moiety. The peptide-conjugated resin having a poly(meth) acrylic acid ester moiety may have a poly(meth) acrylic acid ester moiety, a peptide moiety, and a linker moiety.

In the present specification, " (meth) acrylic" means one or both of "acrylic" and "methacrylic", and " (meth) acrylate" means one or both of "acrylate" and "methacrylate".

<<Polyvinyl alcohol derivative moiety>>

The polyvinyl alcohol derivative moiety (polyvinyl alcohol derivative skeleton) is a structural moiety derived from a polyvinyl alcohol derivative. The polyvinyl alcohol derivative is a compound derived from polyvinyl alcohol.

The peptide-conjugated resin is preferably a peptide-conjugated polyvinyl alcohol derivative having a polyvinyl alcohol derivative moiety and a peptide moiety. The cell scaffold preferably contains a peptide-conjugated polyvinyl alcohol derivative having a polyvinyl alcohol derivative moiety and a peptide moiety. By using the peptide-conjugated polyvinyl alcohol derivative, the effects of the present invention can be more effectively exhibited, and the cell proliferation can be further enhanced.

From the viewpoint of further effectively exhibiting the effects of the present invention and the viewpoint of further enhancing the cell proliferation, the polyvinyl alcohol derivative is preferably a polyvinyl acetal resin, and the polyvinyl alcohol derivative moiety is preferably a polyvinyl acetal resin moiety. That is, the peptide-conjugated resin preferably has a polyvinyl acetal resin moiety and a peptide moiety. The cell scaffold preferably contains a peptide-conjugated polyvinyl acetal resin having a polyvinyl acetal resin moiety and a peptide moiety. Only one kind of each of the polyvinyl alcohol derivative and the polyvinyl acetal resin may be used, or two or more kinds thereof may be used in combination.

The polyvinyl alcohol derivative moiety and the polyvinyl acetal resin moiety preferably have an acetal group, a hydroxyl group, and an acetyl group in side chains. However, the polyvinyl alcohol derivative moiety and the polyvinyl acetal resin moiety may not have, for example, an acetyl group. For example, all of the acetyl groups of the polyvinyl acetal resin moiety are bonded to a linker, so that the polyvinyl alcohol derivative moiety and the polyvinyl acetal resin moiety may not have an acetyl group.

The polyvinyl acetal resin can be synthesized by acetalizing polyvinyl alcohol with an aldehyde.

The aldehyde used for acetalization of polyvinyl alcohol is not particularly limited. Examples of the aldehyde include an aldehyde having 1 to 10 carbon atoms. The aldehyde may or may not have a chain aliphatic group, a cyclic aliphatic group, or an aromatic group. The aldehyde may be a chain aldehyde or a cyclic aldehyde. Only one kind of the aldehyde may be used, or two or more kinds thereof may be used in combination.

From the viewpoint of further enhancing the adhesiveness between the cell scaffold and the cell and the viewpoint of more effectively exhibiting the effects of the present invention, the aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin. From the viewpoint of further enhancing the adhesiveness between the cell scaffold and the cell and the viewpoint of more effectively exhibiting the effects of the present invention, the polyvinyl acetal resin moiety is preferably a polyvinyl butyral resin moiety. The peptide-conjugated resin preferably has a polyvinyl butyral resin moiety and a peptide moiety. The cell scaffold preferably contains a peptide-conjugated polyvinyl butyral resin having a polyvinyl butyral resin moiety and a peptide moiety.

In the peptide-conjugated polyvinyl acetal resin, the acetalization degree of the polyvinyl acetal resin moiety (butyralization degree in the case of the polyvinyl butyral resin moiety) is preferably 40 mol% or more and more preferably 50 mol% or more, and is preferably 90 mol% or less and more preferably 85 mol% or less. When the acetalization degree is the lower limit or more, the peptide-conjugated polyvinyl acetal resin is less likely to swell in the medium. When the acetalization degree is the upper limit or less, solubility in a solvent can be improved.

In the peptide-conjugated polyvinyl acetal resin, the content ratio of the hydroxyl group (hydroxyl group amount) of the polyvinyl acetal resin moiety is preferably 15 mol% or more and more preferably 20 mol% or more, and is preferably 45 mol% or less, more preferably 30 mol% or less, and further preferably 25 mol% or less.

In the peptide-conjugated polyvinyl acetal resin, the acetylation degree (acetyl group amount) of the polyvinyl acetal resin moiety is preferably 1 mol% or more and more preferably 2 mol% or more, and is preferably 5 mol% or less and more preferably 4 mol% or less. When the acetylation degree is the lower limit or more and the upper limit or less, the reaction efficiency between the polyvinyl acetal resin and a linker can be enhanced.

The acetalization degree, the acetylation degree, and the hydroxyl group amount of the polyvinyl acetal resin moiety can be measured by $^1$H-NMR (nuclear magnetic resonance spectrum). The acetalization degree, acetylation degree, and hydroxyl group amount of the polyvinyl acetal resin moiety are each usually mol% with respect to the total sum (100 mol%) of the substance amounts of the respective structural units constituting the peptide-conjugated polyvinyl acetal resin.

<<Poly(meth)acrylic acid ester moiety>>

The poly(meth)acrylic acid ester moiety (poly(meth)acrylic acid ester skeleton) is a structural moiety derived from a poly(meth)acrylic acid ester.

The peptide-conjugated resin preferably has a poly(meth)acrylic acid ester moiety, and is more preferably a peptide-conjugated (meth)acrylic copolymer having a poly(meth)acrylic acid ester moiety and a peptide moiety. The cell scaffold preferably contains a peptide-conjugated (meth)acrylic copolymer having a poly(meth)acrylic acid ester moiety and a peptide moiety. By using the peptide-conjugated (meth)acrylic copolymer, the effects of the present invention can be more effectively exhibited, and the cell proliferation can be further enhanced.

The poly(meth)acrylic acid ester moiety has a skeleton derived from a (meth)acrylic acid ester. The poly(meth) acrylic acid ester is obtained by polymerizing a (meth)acrylic acid ester. Only one kind of the poly (meth) acrylic acid ester may be used, or two or more kinds thereof may be used in combination.

Examples of the (meth)acrylic acid ester include a (meth)acrylic acid alkyl ester, a (meth)acrylic acid cyclic alkyl ester, a (meth)acrylic acid aryl ester, (meth)acrylic acid polyethylene glycols, and (meth)acrylic acid phosphorylcholine. Only one kind of the (meth)acrylic acid ester may be used, or two or more kinds thereof may be used in combination.

Examples of the (meth)acrylic acid alkyl ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate, and isotetradecyl (meth) acrylate.

The (meth)acrylic acid alkyl ester may be substituted with a substituent such as an alkoxy group having 1 to 3 carbon atoms and a tetrahydrofurfuryl group. Examples of such a (meth)acrylic acid alkyl ester include methoxyethyl acrylate and tetrahydrofurfuryl acrylate.

Examples of the (meth)acrylic acid cyclic alkyl ester include cyclohexyl (meth)acrylate and isobornyl (meth) acrylate.

Examples of the (meth)acrylic acid aryl ester include phenyl (meth)acrylate and benzyl (meth)acrylate.

Examples of the (meth)acrylic acid polyethylene glycols include methoxy-polyethylene glycol (meth)acrylate, ethoxy-polyethylene glycol (meth)acrylate, hydroxypolyethylene glycol (meth)acrylate, methoxy-diethylene glycol (meth) acrylate, ethoxy-diethylene glycol (meth)acrylate, hydroxy-diethylene glycol (meth)acrylate, methoxy-triethylene glycol (meth)acrylate, ethoxytriethylene glycol (meth)acrylate, and hydroxy-triethylene glycol (meth)acrylate.

Examples of the (meth)acrylic acid phosphorylcholine include 2-(meth)acryloyloxyethyl phosphorylcholine.

The peptide-conjugated resin having a poly (meth) acrylic acid ester moiety may have a skeleton derived from a monomer other than the (meth)acrylic acid ester.

Examples of the monomer other than the (meth)acrylic acid ester include (meth)acrylamides and a vinyl compound. Only one kind of the monomer other than the (meth)acrylic acid ester may be used, or two or more kinds thereof may be used in combination.

Examples of the (meth)acrylamides include (meth)acrylamide, N-isopropyl (meth)acrylamide, N-tert-butyl (meth)acrylamide, N,N'-dimethyl (meth)acrylamide, (3-(meth)acrylamidepropyl)trimethylammonium chloride, 4-(meth) acryloylmorpholine, 3-(meth)acryloyl-2-oxazolidinone, N-[3-(dimethylamino) propyl] (meth) acrylamide, N-(2-hydroxyethyl) (meth)acrylamide, N-methylol (meth)acrylamide, and 6-(meth)acrylamidohexanoic acid.

Examples of the vinyl compound include ethylene, allylamine, vinylpyrrolidone, maleic anhydride, maleimide, itaconic acid, (meth)acrylic acid, and vinylamine.

<<Peptide moiety>>

The peptide moiety is a structural moiety derived from a peptide. The peptide moiety has an amino acid sequence. The peptide constituting the peptide moiety may be an oligopeptide or a polypeptide. Only one kind of the peptide may be used, or two or more kinds thereof may be used in combination.

The number of amino acid residues in the peptide moiety is preferably 3 or more, more preferably 4 or more, and still more preferably 5 or more, and is preferably 10 or less, more preferably 8 or less, and further preferably 6 or less. When the number of amino acid residues is the lower limit or more and the upper limit or less, the adhesiveness between the cell scaffold and cells after seeding can be further enhanced, and the proliferation rate of cells can be further enhanced. However, the number of amino acid residues in the peptide moiety may be more than 10 or more than 15.

The peptide moiety preferably has a cell adhesive amino acid sequence. The cell adhesive amino acid sequence refers to an amino acid sequence whose cell adhesion activity has been confirmed by a phage display method, a sepharose bead method, or a plate coating method. As the phage display method, for example, the method described in "The Journal of Cell Biology, Volume 130, Number 5, September 1995 1189-1196" can be used. As the sepharose bead method, for example, the method described in "PROTEIN, NUCLEIC ACID, AND ENZYME, Vol. 45, No. 15 (2000) 2477" can be used. As the plate coating method, for example, the method described in "PROTEIN, NUCLEIC ACID, AND ENZYME, Vol. 45, No. 15 (2000) 2477" can be used.

Examples of the cell adhesive amino acid sequence include an RGD sequence (Arg-Gly-Asp), a YIGSR sequence (Tyr-Ile-Gly-Ser-Arg), a PDSGR sequence (Pro-Asp-Ser-Gly-Arg), an HAV sequence (His-Ala-Val), an ADT sequence (Ala-Asp-Thr), a QAV sequence (Gln-Ala-Val), an LDV sequence (Leu-Asp-Val), an IDS sequence (Ile-Asp-Ser), an REDV sequence (Arg-Glu-Asp-Val), an IDAPS sequence (Ile-Asp-Ala-Pro-Ser), a KQAGDV sequence (Lys-Gln-Ala-Gly-Asp-Val), and a TDE sequence (Thr-Asp-Glu). Examples of the cell adhesive amino acid sequence include the sequences described in "Pathophysiology, Vol. 9, No. 7, p. 527 to 535, 1990" and "Journal of Osaka Women's and Children's Hospital, Vol. 8, No. 1, p. 58 to 66, 1992". The peptide moiety may have only one type of the cell adhesive amino acid sequence or two or more types thereof.

The peptide moiety preferably has at least one of the above-described cell adhesive amino acid sequences, more preferably has at least an RGD sequence, a YIGSR sequence, or a PDSGR sequence, still more preferably has an RGD sequence, and particularly preferably has at least an RGD sequence represented by the following Formula (1). In this case, the adhesiveness between the cell scaffold and cells after seeding can be further enhanced, and the proliferation rate of cells can be further enhanced.

$$\text{Arg-Gly-Asp-X} \cdots \qquad \text{Formula (1)}$$

In the above Formula (1), X represents Gly, Ala, Val, Ser, Thr, Phe, Met, Pro, or Asn.

The peptide moiety may be linear or may have a cyclic peptide skeleton. The cyclic peptide skeleton is a cyclic skeleton composed of a plurality of amino acids. From the viewpoint of further effectively exhibiting the effects of the present invention, the cyclic peptide skeleton is preferably composed of four or more amino acids, more preferably composed of five or more amino acids, and preferably composed of ten or less amino acids.

In the peptide-conjugated resin, the content ratio of the peptide moiety is preferably 0.05 mol% or more, more preferably 0.1 mol% or more, further preferably 0.15 mol% or more, and particularly preferably 0.2 mol% or more, and is preferably 25 mol% or less, more preferably 20 mol% or less, further preferably 15 mol% or less, and particularly preferably 10 mol% or less. When the content ratio of the peptide moiety is the lower limit or more, the adhesiveness between the cell scaffold and cells after seeding can be further enhanced, and the proliferation rate of cells can be further enhanced. When the content ratio of the peptide moiety is the upper limit or less, the production cost can be suppressed.

The content ratio (mol%) of the peptide moiety is the amount of substance of the peptide moiety with respect to the total of the amount of substance of structural units constituting the peptide-conjugated resin.

The content ratio of the peptide moiety can be measured by, for example, nuclear magnetic resonance (NMR).

<<Linker moiety>>

The linker moiety is a structural moiety derived from a linker. The linker moiety is usually located between the polyvinyl alcohol derivative moiety or the poly (meth) acrylic acid ester moiety and the peptide moiety. The polyvinyl alcohol derivative moiety or the poly(meth)acrylic acid ester moiety and the peptide moiety are bonded to each other via the linker moiety. The linker moiety is formed by a linker (crosslinking agent). Only one kind of the linker may be used, or two or more kinds thereof may be used in combination.

The linker is preferably a compound having a functional group capable of bonding to the peptide, and more preferably a compound having a functional group capable of condensing with a carboxyl group or an amino group of the peptide.

Examples of the functional group capable of condensing with a carboxyl group or an amino group of the peptide include a carboxyl group, a thiol group, an amino group, a hydroxyl group, and a cyano group.

From the viewpoint of favorably reacting with the peptide, the linker is preferably a compound having a carboxyl group or an amino group, and more preferably a compound having a carboxyl group.

In the case of obtaining a peptide-conjugated resin having a polyvinyl alcohol derivative moiety, examples of the linker having a carboxyl group include (meth)acrylic acid and carboxyl group-containing acrylamide. By using a carboxylic acid (carboxylic acid monomer) having a polymerizable unsaturated group as the linker having a carboxyl group, the carboxylic acid monomer can be polymerized by graft polymerization at the time of introduction of the linker, so that the number of carboxyl groups that can react with the peptide can be increased.

From the viewpoint of bonding the polyvinyl alcohol derivative and the peptide well, the linker is preferably (meth) acrylic acid, and more preferably acrylic acid.

In the case of obtaining a peptide-conjugated resin having a poly(meth)acrylic acid ester moiety, the linker preferably has a functional group capable of bonding to a (meth)acrylic acid ester. Examples of the functional group capable of bonding to a (meth)acrylic acid ester include a vinyl group, a (meth)acryloyl group, and an allyl group. The linker more preferably has a (meth)acryloyl group as the functional group capable of bonding to a (meth)acrylic acid ester, and is preferably a compound having a carboxyl group or an amino group and having a (meth)acryloyl group.

Examples of the linker in the case of obtaining a peptide-conjugated resin having a poly(meth)acrylic acid ester moiety include (meth)acrylic acid, itaconic acid, and acrylamide.

From the viewpoint of bonding the poly (meth) acrylic acid ester and the peptide well, the linker is preferably (meth)acrylic acid or itaconic acid, and more preferably (meth)acrylic acid.

<Other details of cell scaffold>

The number average molecular weight of the peptide-conjugated resin is preferably 10000 or more, more preferably 50000 or more, and further preferably 100000 or more, and is preferably 5000000 or less, more preferably 2500000 or less, and further preferably 1000000 or less. When the number average molecular weight is the lower limit or more, the cell scaffold is less likely to be eluted into the liquid medium during cell culture, and the cell scaffold is less likely to be detached from the substrate or the like during cell culture. When the number average molecular weight is the upper limit or less, the solubility in an alcohol solvent can be enhanced.

The number average molecular weight of the peptide-conjugated resin can be measured by, for example, the following method. The peptide-conjugated resin is dissolved in tetrahydrofuran (THF) to prepare a 0.2 wt% solution of the peptide-conjugated resin. Next, evaluation is performed under the following measurement conditions using a gel permeation chromatography (GPC) measuring device (APC system, manufactured by Waters Corporation).

Column: HSPgel HR MB-M 6.0 $\times$ 150 mm
Flow rate: 0.5 mL/min
Column temperature: 40°C
Injection volume: 10 $\mu$L
Detector: RI, PDA
Standard sample: Polystyrene

The cell scaffold may contain a synthetic resin having no peptide moiety (synthetic resin to which no peptide is bonded) and a peptide-conjugated resin. The cell scaffold may contain a synthetic resin having the polyvinyl alcohol derivative moiety or the poly(meth)acrylic acid ester moiety and having no peptide moiety, and a peptide-conjugated resin. The cell scaffold may contain other components such as a resin having neither the polyvinyl alcohol derivative moiety nor the poly(meth)acrylic acid ester moiety. Examples of the other components include polyolefin resins, polyether resins, polyesters, epoxy resins, polyamide resins, polyimide resins, polyurethane resins, polycarbonate resins, celluloses, and polypeptides. Only one kind of the other components may be used, or two or more kinds thereof may be used in combination.

It is preferable that the cell scaffold contains the peptide-conjugated resin and does not contain or contains a synthetic resin to which no peptide is bonded.

The cell scaffold preferably has a layer containing the peptide-conjugated resin. The cell scaffold may have only a layer containing the peptide-conjugated resin, and may have a layer containing the peptide-conjugated resin and a layer containing a synthetic resin to which no peptide is bonded. From the viewpoint of more effectively exhibiting the effects of the present invention and the viewpoint of further enhancing the cell proliferation, a first surface of the layer containing the peptide-conjugated resin is preferably an upper surface of the cell scaffold. The peptide-conjugated resin is preferably present at least on the upper surface of the cell scaffold.

The content of the peptide-conjugated resin in 100 wt% of the cell scaffold is preferably 90 wt% or more, more preferably 95 wt% or more, further preferably 97.5 wt% or more, particularly preferably 99 wt% or more, and most preferably 100 wt% (total amount). When the content of the peptide-conjugated resin is the lower limit or more, the effects of the present invention can be more effectively exhibited. The content of the peptide-conjugated resin in 100 wt% of the cell scaffold may be 100 wt% or less or less than 100 wt%.

The total of the content of the peptide-conjugated resin and the content of the synthetic resin to which no peptide is bonded in 100 wt% of the cell scaffold is preferably 90 wt% or more, more preferably 95 wt% or more, further preferably 97.5 wt% or more, particularly preferably 99 wt% or more, and most preferably 100 wt% (total amount). When the total content is the lower limit or more, the effects of the present invention can be more effectively exhibited. The total of the content of the peptide-conjugated resin and the content of the synthetic resin to which no peptide is bonded in 100 wt% of the cell scaffold may be 100 wt% or less or less than 100 wt%.

The cell scaffold preferably has a layer containing the cell adhesive protein. The cell scaffold may have only a layer containing the cell adhesive protein, and may have a layer containing the cell adhesive protein and a layer containing no cell adhesive protein. From the viewpoint of more effectively exhibiting the effects of the present invention and the viewpoint of further enhancing the cell proliferation, a first surface of the layer containing the cell adhesive protein is preferably an upper surface of the cell scaffold. The cell adhesive protein is preferably present at least on the upper surface of the cell scaffold.

The content of the cell adhesive protein in 100 wt% of the cell scaffold is preferably 90 wt% or more, more preferably 95 wt% or more, further preferably 97.5 wt% or more, particularly preferably 99 wt% or more, and most preferably 100 wt% (total amount). When the content of the cell adhesive protein is the lower limit or more, the effects of the present invention can be more effectively exhibited. The content of the cell adhesive protein in 100 wt% of the cell scaffold may be 100 wt% or less or less than 100 wt%.

(Other details of cell culture substrate)

In the cell culture substrate, a cell scaffold is disposed in a dot pattern on the first surface of the substrate. In the cell culture substrate, a region where the cell scaffold is not disposed on the first surface of the substrate preferably has a region subjected to a cell low adhesion treatment. In this case, the efficiency of inducing differentiation from pluripotent stem cells into hematopoietic progenitor cells can be further enhanced. The reason for this is not clear, but the following possibility is considered: the possibility that the extension of endothelial cells to the region subjected to the cell low adhesion treatment is effectively suppressed, and the endothelial cells are localized on the cell scaffold, thereby promoting the induction of differentiation into hematopoietic progenitor cells; and the possibility of promoting the induction of differentiation into hematopoietic progenitor cells by effectively suppressing the adsorption of the supplement in the liquid medium to the substrate. However, the above is presumptive, and the reason why the efficiency of inducing differentiation can be further enhanced by having the region subjected to a cell low adhesion treatment in the substrate is not limited thereto.

Examples of the cell low adhesion treatment include a coating treatment with a fluorine-based polymer, an amphoteric polymer, an MPC polymer, a hydrogel, or the like. Examples of commercially available products of the substrate subjected to such a cell low adhesion treatment include a low adhesion treatment container and an ultra-low adhesion treatment container.

From the viewpoint of further enhancing the efficiency of inducing differentiation, the region subjected to a cell low adhesion treatment is preferably a region coated with an MPC polymer or a hydrogel.

Note that the region where the cell scaffold is disposed on the first surface of the substrate may be subjected to a cell low adhesion treatment or may not be subjected to a cell low adhesion treatment.

The cell culture substrate including a cell scaffold containing a peptide-conjugated resin can be prepared, for example, by the following method (A) or (B). The following (A) is an example of a method for preparing a cell culture substrate by using a method of synthesizing a peptide-conjugated resin by a liquid phase method, and the following (B) is an example of a method for preparing a cell culture substrate by using a method of synthesizing a peptide-conjugated resin by a solid phase method.

(A) A solution containing a synthetic resin (hereinafter, referred to as "synthetic resin X") having a functional group capable of reacting with an amino group or a functional group capable of reacting with a carboxyl group, and a peptide is prepared. The synthetic resin X is reacted with the peptide to obtain a peptide-conjugated resin. A solution containing the peptide-conjugated resin and a solvent is applied in a dot pattern onto the first surface of the substrate, and then the solvent is volatilized.

(B) A solution containing a synthetic resin (synthetic resin X) having a functional group capable of reacting with an amino group or a functional group capable of reacting with a carboxyl group is prepared. The solution containing the synthetic resin X is applied in a dot pattern onto the first surface of the substrate. A liquid containing a peptide is added onto the first surface of the substrate, and the synthetic resin X is reacted with the peptide.

Examples of the synthetic resin X include a (meth)acrylate polymer and a polyvinyl acetal derivative to which the linker is bonded. Examples of the method for applying the solution onto the first surface of the substrate in a dot pattern include a method using a dispenser, a method using an inkjet device, a method using a stamp, and a method using a pipetman.

The cell culture substrate including a cell scaffold containing a cell adhesive protein can be prepared, for example, by applying a solution containing a cell adhesive protein in a dot pattern onto the first surface of the substrate and then volatilizing a solvent.

[Method for producing hematopoietic progenitor cells and method for producing leukocytes]

(Cell cluster forming step)

The method for producing hematopoietic progenitor cells and the method for producing leukocytes according to the present invention includes a step of culturing pluripotent stem cells using the cell culture substrate to form a cell cluster on the surface of the cell scaffold (cell cluster forming step).

Examples of the pluripotent stem cells include induced pluripotent stem cells (iPS cells), embryonic stem cells (ES cells), and embryonic germ stem cells (EG cells).

From the viewpoint of application to the regenerative medicine field and the drug discovery field, the pluripotent stem cells are preferably iPS cells.

The cell cluster forming step preferably includes the following steps (a), (b), and (c) in this order.

Step (a): a step of seeding pluripotent stem cells on the cell culture substrate.

Step (b): a step of culturing the pluripotent stem cells.

Step (c): a step of forming a cell cluster on a surface of the cell scaffold.

<Step (a)>

In step (a), the maintenance cultured pluripotent stem cells are preferably seeded on the cell culture substrate.

The seeding density of the pluripotent stem cells in step (a) may be 5000 cells or more, 10000 cells or more, or 15000 cells or more, and may be 80000 cells or less, 65000 cells or less, or 50000 cells or less, with respect to an area of 1 $cm^2$ of the first surface of the substrate. In the present invention, even when the seeding density is relatively high or the seeding density is relatively low, hematopoietic progenitor cells or leukocytes can be stably obtained.

<Step (b)>

In step (b), the pluripotent stem cells seeded in step (a) are cultured. As a medium for culturing the pluripotent stem cells in step (b), a conventionally known medium used for culturing pluripotent stem cells can be used. The medium is preferably a liquid medium. As the medium, a commercially available medium may be used, or a self-prepared medium may be used. Examples of commercially available products of the medium include "Stem Fit AK03N" manufactured by Ajinomoto Co., Inc., "Stem Fit AK02N" manufactured by Ajinomoto Co., Inc., "TeSR-E8", "mTeSR1", and "mTeSR Plus" manufactured by STEMCELL Technologies, and "StemFlex" and "Essential 8" manufactured by Gibco. As the medium, a medium containing a ROCK (Rho binding kinase) specific inhibitor may be used, a medium not containing a ROCK specific inhibitor may be used, and a medium containing ROCK specific inhibitor and a medium not containing a ROCK specific inhibitor may be selectively used according to the culture time.

The culture temperature in step (b) is preferably 35°C or higher and preferably 38°C or lower. The $CO_2$ concentration during culture in step (b) is preferably 4% or more and preferably 6% or less. For example, the culture conditions in step (b) are conditions of 37°C and a $CO_2$ concentration of 5%. The culture time in step (b) can be, for example, 2 days to 8 days (preferably 4 days). During the culture in step (b), the medium may or may not be replaced. When the medium is replaced, for example, the medium can be replaced with a fresh medium at a frequency of once a day.

<Step (c)>

In step (c), a cell cluster can be obtained from the pluripotent stem cells. By performing step (b), a cell cluster is formed on the surface of the cell scaffold. More specifically, by performing step (b), a cell cluster is formed on the upper surface of the cell scaffold. The cell cluster is preferably a cell cluster containing pluripotent stem cells. In step (c), usually, a cell cluster corresponding to the size of the upper surface of the cell scaffold is formed.

(Differentiation induction step)

The method for producing hematopoietic progenitor cells according to the present invention includes a step of inducing differentiation of the obtained cell cluster into a cell population containing hematopoietic progenitor cells (cell group containing hematopoietic progenitor cells) (differentiation induction step).

The method for producing leukocytes according to the present invention includes a step of inducing differentiation of the obtained cell cluster into a cell population containing monocytes, lymphocytes, or granulocytes (cell group containing monocytes, lymphocytes, or granulocytes) (differentiation induction step). The differentiation induction step in the method for producing leukocytes according to the present invention may be a step of differentiating into a cell population containing monocytes, a step of differentiating into a cell population containing lymphocytes, or a step of differentiating into a cell population containing granulocytes.

In the present specification, the common configuration between the differentiation induction step in the method for producing hematopoietic progenitor cells and the differentiation induction step in the method for producing leukocytes will be simply described as "differentiation induction step".

In the differentiation induction step, differentiation induction is performed using the cell cluster obtained in the cell cluster forming step.

As differentiation inducing conditions and culture conditions in the differentiation induction step, conventionally known differentiation inducing conditions and culture conditions for differentiating pluripotent stem cells into hematopoietic progenitor cells or leukocytes can be employed. In the differentiation induction step, the same cell culture substrate as the cell culture substrate used in the cell cluster forming step may be used, or a cell culture substrate different from the cell culture substrate used in the cell cluster forming step may be used. From the viewpoint of inducing differentiation without peeling the obtained cell cluster from the cell culture substrate, it is preferable to use the same cell culture substrate as the cell culture substrate used in the cell cluster forming step at least at the start time of the differentiation induction step. From the viewpoint of inducing differentiation without peeling the cells before or during differentiation from the cell culture substrate, it is preferable to use the same cell culture substrate as the cell culture substrate used in the cell cluster forming step at all time points of the differentiation induction step.

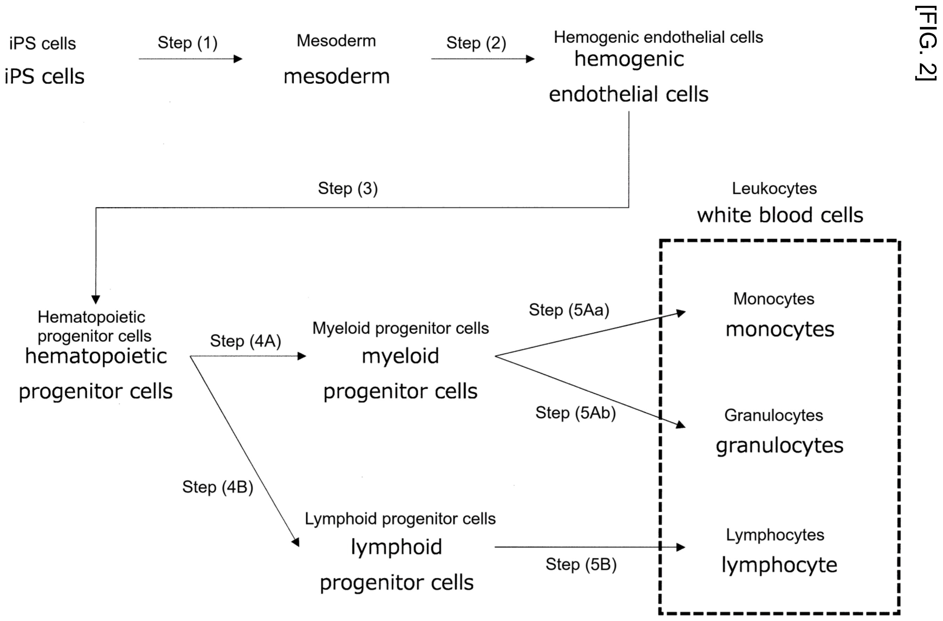

Fig. 2 shows an example of a flow of inducing differentiation of leukocytes (monocytes, lymphocytes, or granulocytes) from iPS cells via hematopoietic progenitor cells. In each step of the differentiation induction step, an example of a usable liquid medium, an example of a usable differentiation-inducing factor, and a reference of the culture period are shown in Tables 1 to 3. Table 4 shows the formal names of differentiation-inducing factors. Note that, in Fig. 2 and Tables 1 to 3, the names of the steps (steps (1) to (5B)) correspond to the names of the steps described later.

[Table 1]

|  | Step (1) | Step (2) | Step (3) |
|---|---|---|---|
| Example of usable liquid medium | Manufactured by Gibco "Essential-8 medium" | Manufactured by Gibco "Essential-6 medium" | Manufactured by Gibco "StemPro-34 medium" |
| Example of usable differentiation-inducing factor | VEGF BMP4 GSK-3 inhibitor | VEGF FGF-2 SCF ALK5 inhibitor | VEGF SCF TPO IL-3 FL |
| Culture period (days) | 1 to 4 | 1 to 4 | 2 to 5 |
| Cells induced to differentiate | Mesoderm | Hemogenic endothelial cells | Hematopoietic progenitor cells |

[Table 2]

|  | Step (4A) | Step (5Aa) | Step (5Ab) |
|---|---|---|---|
| Example of usable liquid medium | Manufactured by Gibco "StemPro-34 medium" | Manufactured by Gibco "StemPro-34 medium" | Manufactured by Sigma-Aldrich Co., Ltd. "IMDM medium" |
| Example of usable differentiation-inducing factor | SCF TPO IL-3 FL M-CSF | FL M-CSF GM-CSF | TPO SCF FLT3-L G-CSF |
| Culture period (days) | 2 to 5 | 3 to 60 | 17 to 30 |

(continued)

|  | Step (4A) | Step (5Aa) | Step (5Ab) |
|---|---|---|---|
| Cells induced to differentiate | Myeloid progenitor cells | Monocytes | Granulocytes |

[Table 3]

|  | Step (4B) | Step (5B) |
|---|---|---|
| Example of usable liquid medium | Manufactured by VERITAS Corporation "StemSpan SFEM II" Manufactured by ThermoFisher "αMEM + BIT" "IMDM + BIT" | Manufactured by VERITAS Corporation "StemSpan SFEM II" |
| Example of usable differentiation-inducing factor | DL4 SCF TPO IL-7 FLT3L SDF-1α SB203580 | CD3 monoclonal antibody IL-7 IL-2 Dexamethasone |
| Culture period (days) | 10 to 28 | 30 to 50 |
| Cells induced to differentiate | Lymphoid progenitor cells | Lymphocytes (CD8 T cells) |

[Table 4]

| Abbreviation | Formal name |
|---|---|
| BMP4 | Bone morphogenetic protein-4 |
| DL4 | Delta-like4 |
| FGF-2 | Fibroblast growth factor 2 |
| bFGF | Basic fibroblast growth factor |
| FL | FMS-like tyrosine kinase 3 ligand |
| FLT3-L | FMS-related tyrosine kinase 3 ligand |
| G-CSF | Granulocyte-colony stimulating factor |
| GM-CSF | Granulocyte-macrophage colony-stimulating factor |
| GSK-3 | Glycogen synthase kinase 3 |
| IL-2 | Interleukin-2 |
| IL-3 | Interleukin-3 |
| IL-7 | Interleukin-7 |
| M-CSF | Macrophage colony-stimulating factor |
| SCF | Stem cell factor |
| SDF-1α | Stromal cell derived factor 1α |
| TPO | Thrombopoietin |
| VEGF | Vascular endothelial growth factor |

In the method for producing hematopoietic progenitor cells, the differentiation induction step preferably includes the following steps (1), (2), and (3) in this order.

Step (1): a step of culturing the obtained cell cluster in the presence of a medium containing a differentiation-inducing factor to obtain a cell population containing mesoderm.

Step (2): a step of culturing the obtained cell population containing mesoderm in the presence of a medium containing a differentiation-inducing factor to obtain a cell population containing hemogenic endothelial cells.

Step (3): a step of culturing the obtained cell population containing hemogenic endothelial cells in the presence of a medium containing a differentiation-inducing factor to obtain a cell population containing hematopoietic progenitor cells.

<Step (1)>

In step (1), the cell cluster obtained in the cell cluster forming step is cultured. Step (1) is preferably a step of culturing the obtained cell cluster in a state of adhering to the surface of the cell scaffold in the presence of a medium containing a differentiation-inducing factor to obtain a cell population containing mesoderm. Step (1) is preferably a step of inducing differentiation of pluripotent stem cells contained in the cell cluster into a cell population containing mesoderm.

<Step (2)>

In step (2), the cell population containing mesoderm obtained in step (1) is cultured. Step (2) is preferably a step of culturing the obtained cell population containing mesoderm in a state of adhering to the surface of the cell scaffold in the presence of a medium containing a differentiation-inducing factor to obtain a cell population containing hemogenic endothelial cells. Step (2) is preferably a step of inducing differentiation of mesoderm into a cell population containing hemogenic endothelial cells.

<Step (3)>

In step (3), the cell population containing hemogenic endothelial cells obtained in step (2) is cultured. Step (3) is preferably a step of culturing the obtained cell population containing hemogenic endothelial cells in a state of adhering to the surface of the cell scaffold in the presence of a medium containing a differentiation-inducing factor to obtain a cell population containing hematopoietic progenitor cells. Step (3) is preferably a step of inducing differentiation of hemogenic endothelial cells into a cell population containing hematopoietic progenitor cells.

Whether or not the cells have been induced into hematopoietic progenitor cells can be confirmed, for example, by observing the morphology of the cells or analyzing whether or not a differentiation marker is expressed in the cells by a flow cytometer. As the differentiation markers of hematopoietic progenitor cells, CD34, CD43, and the like are known. For example, co-expression of CD34 and CD43 can confirm induction to hematopoietic progenitor cells.

The culture temperature in each of steps (1), (2), and (3) is preferably 35°C or higher and preferably 38°C or lower. The $CO_2$ concentration during culture in each of steps (1), (2), and (3) is preferably 4% or more and preferably 6% or less. For example, the culture conditions in each of steps (1), (2), and (3) are conditions of 37°C and a $CO_2$ concentration of 5%. The culture time in each of steps (1), (2), and (3) can be set to, for example, 4 days to 13 days in total. During the culture in each of steps (1), (2), and (3), the medium may or may not be replaced. When the medium is replaced, for example, the medium can be replaced with a fresh medium at a frequency of once every two days.

Hematopoietic progenitor cells are induced in about 7 days, for example, from the start of culture in step (1), and a cell population containing hematopoietic progenitor cells can be obtained. The obtained cell population containing hematopoietic progenitor cells can be induced to differentiate into a cell population containing various blood cells under conventionally known differentiation inducing conditions and culture conditions. For example, leukocytes can be produced by inducing differentiation of the obtained cell population containing hematopoietic progenitor cells into a cell population containing monocytes, lymphocytes, or granulocytes.

In the method for producing leukocytes, the differentiation induction step preferably includes the following step (4A) or step (4B) in addition to steps (1), (2), and (3). In the method for producing leukocytes, the differentiation induction step may include the above-described step (1), step (2), and step (3) and the following step (4A) in this order, and may include the above-described step (1), step (2), and step (3) and the following step (4B) in this order.

Step (4A): a step of culturing the obtained cell population containing hematopoietic progenitor cells in the presence of a medium containing a differentiation-inducing factor to obtain a cell population containing myeloid progenitor cells.

Step (4B): a step of culturing the obtained cell population containing hematopoietic progenitor cells in the presence of a medium containing a differentiation-inducing factor to obtain a cell population containing lymphoid progenitor cells.

<Step (4A)>

In step (4A), the cell population containing hematopoietic progenitor cells obtained in step (3) is cultured. Step (4A) may be a step of culturing the obtained cell population containing hematopoietic progenitor cells in a state of adhering to the surface of the cell scaffold in the presence of a medium containing a differentiation-inducing factor to obtain a cell population containing myeloid progenitor cells. Step (4A) is preferably a step of inducing differentiation of hematopoietic progenitor cells into a cell population containing myeloid progenitor cells.

<Step (4B)>

In step (4B), the cell population containing hematopoietic progenitor cells obtained in step (3) is cultured. Step (4B) preferably includes a step of recovering floating cells in the obtained cell population containing hematopoietic progenitor cells and a step of culturing the recovered cells in the presence of a medium containing a differentiation-inducing factor to obtain a cell population containing lymphoid progenitor cells. In this case, the efficiency of inducing differentiation into lymphocytes can be further enhanced, and in particular, the efficiency of inducing differentiation into T cells can be further enhanced. Step (4B) is preferably a step of inducing differentiation of hematopoietic progenitor cells into a cell population containing lymphoid progenitor cells.

The method for producing leukocytes preferably includes a step of inducing differentiation of a cell population containing hematopoietic progenitor cells obtained by the above-described method for producing hematopoietic progenitor cells into a cell population containing monocytes, lymphocytes, or granulocytes. In the method for producing leukocytes, it is preferable that leukocytes containing at least one of monocytes, lymphocytes, and granulocytes are produced. The method for producing leukocytes is preferably any one of a method for producing monocytes, a method for producing granulocytes, and a method for producing lymphocytes. The method for producing leukocytes may be a method for producing monocytes, a method for producing granulocytes, or a method for producing lymphocytes.

When the method for producing leukocytes is a method for producing monocytes, the differentiation induction step preferably includes the above-described step (4A) and the following step (5Aa). That is, the differentiation induction step preferably includes the above-described step (1), step (2), step (3), and step (4A) and the following step (5Aa) in this order.

Step (5Aa): a step of culturing the obtained cell population containing myeloid progenitor cells in the presence of a medium containing a differentiation-inducing factor to obtain a cell population containing monocytes.

<Step (5Aa)>

In step (5Aa), it is preferable to culture the cell population containing myeloid progenitor cells obtained in step (4A). Step (5Aa) may be a step of culturing the obtained cell population containing myeloid progenitor cells in a state of adhering to the surface of the cell scaffold in the presence of a medium containing a differentiation-inducing factor to obtain a cell population containing monocytes. Step (5Aa) is preferably a step of inducing differentiation of myeloid progenitor cells into a cell population containing monocytes.

Whether or not the cells have been induced into monocytes can be confirmed, for example, by observing the morphology of the cells or analyzing whether or not a differentiation marker is expressed in the cells by a flow cytometer. As the differentiation markers of monocytes, CD14 and the like are known.

It is also possible to differentiate the obtained monocytes into a cell population containing phagocytic cells such as dendritic cells and macrophages. The method for producing leukocytes may further include a step of culturing the obtained cell population containing monocytes in the presence of a medium containing a differentiation-inducing factor to obtain a cell population containing phagocytic cells.

When the method for producing leukocytes is a method for producing granulocytes, the differentiation induction step preferably includes the above-described step (4A) and the following step (5Ab). That is, the differentiation induction step preferably includes the above-described step (1), step (2), step (3), and step (4A) and the following step (5Ab) in this order.

Step (5Ab): a step of culturing the obtained cell population containing myeloid progenitor cells in the presence of a medium containing a differentiation-inducing factor to obtain a cell population containing granulocytes.

<Step (5Ab)>

In step (5Ab), it is preferable to culture the cell population containing myeloid progenitor cells obtained in step (4A). Step (5Ab) may be a step of culturing the obtained cell population containing myeloid progenitor cells in a state of adhering to the surface of the cell scaffold in the presence of a medium containing a differentiation-inducing factor to obtain a cell population containing granulocytes. Step (5Ab) is preferably a step of inducing differentiation of myeloid progenitor cells into a cell population containing granulocytes. The granulocyte is preferably at least one of neutrophils, eosinophils, and basophils.

Whether or not the cells have been induced into granulocytes can be confirmed, for example, by observing the morphology of the cells or analyzing whether or not a differentiation marker is expressed in the cells by a flow cytometer. As the differentiation markers of granulocytes, CD15, CD62L, and the like are known.

When the method for producing leukocytes is a method for producing lymphocytes, the differentiation induction step preferably includes the above-described step (4B) and the following step (5B). That is, the differentiation induction step preferably includes the above-described step (1), step (2), step (3), and step (4B) and the following step (5B) in this order.

Step (5B): a step of culturing the obtained cell population containing lymphoid progenitor cells in the presence of a medium containing a differentiation-inducing factor to obtain a cell population containing lymphocytes.

<Step (5B)>

In step (5B), it is preferable to culture the cell population containing lymphoid progenitor cells obtained in step (4B). Step (5B) is preferably a step of inducing differentiation of lymphoid progenitor cells into a cell population containing lymphocytes. The lymphocyte is preferably at least one of B cells, T cells, and NK cells. When a cell population containing T cells is obtained, step (5B) is preferably performed in the presence of a Notch ligand.

Whether or not the cells have been induced into lymphocytes can be confirmed, for example, by observing the morphology of the cells or analyzing whether or not a differentiation marker is expressed in the cells by a flow cytometer. As the differentiation markers of lymphocytes, CD3, CD4, CD8 (T cell line differentiation marker), CD56 (NK cell line differentiation marker), and the like are known.

Hereinafter, the present invention will be specifically described with reference to Examples. The present invention is not limited to the following Examples.

(Example 1)

(1) Preparation of cell culture substrate

As a substrate (cell culture substrate body), a 12-well plate (manufactured by Corning Incorporated, surface treatment: tissue culture treatment (TC treatment), bottom area of each well: 3.8 cm$^2$, described as 12wP in the table) was prepared. As a polyvinyl acetal resin, a polyvinyl butyral resin having an acetalization degree (butyralization degree) of 65 mol%, a hydroxyl group amount of 32 mol%, and an acetyl group amount of 3 mol% was prepared. Acrylic acid (30 parts by weight) and a polyvinyl acetal resin (70 parts by weight) were dissolved in 255 parts by weight of tetrahydrofuran to obtain a polymer mixed solution. PERBUTYL O (0.015 parts by weight) (manufactured by NOF CORPORATION) was dissolved in the obtained polymer mixed solution, and the mixture was reacted at 90°C for 6 hours. Next, the solution after the reaction was mixed with 30000 parts by weight of water. The obtained precipitate was vacuum-dried at 80°C for 3 hours to prepare a polyvinyl acetal resin having a linker (acrylic acid) (polyvinyl butyral resin having a linker). Hereinafter, the "polyvinyl acetal resin having a linker" may be referred to as the "polyvinyl acetal resin (X)".

The obtained polyvinyl acetal resin (X) and ethanol were mixed. The obtained mixed liquid was applied in a dot pattern on the first surface of the substrate using a dispenser, and then dried to volatilize the solvent.

A cyclic peptide having an amino acid sequence of Arg-Gly-Asp-Phe-Lys (five amino acid residues, forming a cyclic skeleton by bonding Arg and Lys, Phe is a D form, c-RGDfK) was prepared. The peptide (10 parts by weight) and 1-ethyl-3-(3-dimethylaminopropyl)carbodiimide hydrochloride (condensing agent) (10 parts by weight) were added to 80 parts by weight of methanol to prepare a peptide-conjugated liquid. The peptide-conjugated liquid was added onto the first surface of the substrate and was reacted at 30°C, and a carboxyl group in the structural unit derived from acrylic acid of the polyvinyl acetal resin (X) and an amino group of Lys of the peptide were dehydrated and condensed.

In this way, a cell culture substrate including a substrate and a cell scaffold disposed in a dot pattern on the first surface of the substrate was obtained. The cell scaffold contains a peptide-conjugated polyvinyl acetal resin (peptide-conjugated resin).

The details of the obtained cell culture substrate are shown in the following table. Note that the cell scaffold prepared as described above was described as "PVB-AA (composition 1)" in the following table.

(2) Culture of cells

Media A1 to E1 for differentiation induction shown in the following table were prepared.

[Table 5]

| | Basal medium | Added differentiation-inducing factor and concentration |
|---|---|---|
| Medium A1 | Essential-8 medium (Gibco) | VEGF (manufactured by R&D Systems, Inc.) 160 ng/mL<br>BMP4 (manufactured by Miltenyi Biotec) 160 ng/mL<br>CHIR99021 (GSK-3 inhibitor, manufactured by Miltenyi Biotec) 8 $\mu$M |
| Medium B1 | Essential-6 medium (Gibco) | VEGF (manufactured by R&D Systems, Inc.) 160 ng/ml<br>FGF-2 (manufactured by Miltenyi Biotec) 100 ng/mL<br>SCF (manufactured by Miltenyi Biotec) 100 ng/mL<br>SB431542 (ALK5 inhibitor, manufactured by Miltenyi Biotec) 4 $\mu$M |
| Medium C1 | StemPro-34 medium (Gibco) | VEGF (manufactured by R&D Systems, Inc.) 80 ng/mL<br>SCF (manufactured by Miltenyi Biotec) 100 ng/mL<br>TPO (manufactured by Miltenyi Biotec) 20 ng/mL<br>IL-3 (manufactured by Miltenyi Biotec) 100 ng/mL<br>FL (manufactured by Miltenyi Biotec) 100 ng/ mL |
| Medium D1 | StemPro-34 medium (Gibco) | SCF (manufactured by Miltenyi Biotec) 100 ng/mL<br>TPO (manufactured by Miltenyi Biotec) 20 ng/mL<br>IL-3 (manufactured by Miltenyi Biotec) 100 ng/mL<br>FL (manufactured by Miltenyi Biotec) 100 ng/mL<br>M-CSF (manufactured by Miltenyi Biotec) 100 ng/mL |
| Medium E1 | StemPro-34 medium (Gibco) | FL (manufactured by Miltenyi Biotec) 100 ng/mL<br>M-CSF (manufactured by Miltenyi Biotec) 100 ng/mL<br>GM-CSF (manufactured by Miltenyi Biotec) 50 ng/mL |

(2-1) Maintenance culture of iPS cells

As iPS cells, an iPS cell line (053018A line, described as line 1 in the following table) established from human fibroblasts by The Kyoto University iPS Cell Research Foundation was used. The iPS cells were seeded on a 6-well plate coated with laminin on the entire bottom surface, and maintenance cultured using StemFit AK03N medium (manufactured by Ajinomoto Inc.) under the conditions of 37°C and a $CO_2$ concentration of 5%.

(2-2) Culture of iPS cells (cell cluster forming step)

The maintenance cultured iPS cells were detached from the 6-well plate at 37°C for 10 minutes using a release agent ("TrypLE Select" manufactured by Thermo Fisher Scientific). The detached iPS cells were diluted with a StemFit AK03N medium containing 10$\mu$M of a ROCK inhibitor (Y-27632), and then 1.5 mL of the iPS cells were seeded on the prepared cell culture substrate at a seeding density of $8.0 \times 10^4$ cells/well. Then, the cells were cultured in the presence of StemFit AK03N medium containing 10 $\mu$M ROCK inhibitor (Y-27632) at 37°C and a $CO_2$ concentration of 5%. The day after seeding, the medium was changed to StemFit AK03N medium containing no ROCK inhibitor (Y-27632). Thereafter, the cells were further cultured for 3 days under the conditions of 37°C and a $CO_2$ concentration of 5% to form a cell cluster (cell cluster containing iPS cells) on the upper surface of the cell scaffold in the cell culture substrate.

(2-3) Differentiation induction step (step (1), Day 0 to Day 2)

The obtained cell cluster containing iPS cells was washed with 1.6 mL of Essential-8 medium. Next, 1.6 mL of the medium A1 was added into the cell culture substrate, and the cells were cultured for 2 days under the conditions of 37°C and a $CO_2$ concentration of 5% in a state in which the cell cluster adhered onto the surface of the cell scaffold. In this way, a cell population including mesoderm was obtained.

(2-4) Differentiation induction step (step (2), Day 2 to Day 4)

The medium A1 in the cell culture substrate was removed, 1.6 mL of the medium B1 was added into the cell culture substrate, and the cells were cultured for 2 days under the conditions of 37°C and a $CO_2$ concentration of 5% in a state in which the cell population containing mesoderm adhered onto the surface of the cell scaffold. In this way, a cell population including hemogenic endothelial cells was obtained.

(2-5) Differentiation induction step (step (3), Day 4 to Day 7)

The medium B1 in the cell culture substrate was removed, 3.2 mL of the medium C1 was added into the cell culture substrate, and the cells were cultured for 3 days under the conditions of 37°C and a $CO_2$ concentration of 5% in a state in which the cell population containing hemogenic endothelial cells adhered onto the surface of the cell scaffold. In this way, a cell population containing hematopoietic progenitor cells was obtained.

(2-6) Differentiation induction step (step (4A), Day 7 to Day 10)

The medium C1 in the cell culture substrate was removed, 2 mL of the medium D1 was added into the cell culture substrate, and the cells were cultured for 3 days under the conditions of 37°C and a $CO_2$ concentration of 5% in a state in which the cell population containing hematopoietic progenitor cells adhered onto the surface of the cell scaffold. In this way, a cell population containing myeloid progenitor cells was obtained.

(2-7) Differentiation induction step (step (5Aa), Day 10 to Day 15)

The medium D1 in the cell culture substrate was removed, 1.6 mL of the medium E1 was added into the cell culture substrate, and the cells were cultured under the conditions of 37°C and a $CO_2$ concentration of 5% in a state in which the cell population containing myeloid progenitor cells adhered onto the surface of the cell scaffold. During culture, half of the liquid in the cell culture substrate was replaced with a fresh medium E1 at a frequency of once every two days. Note that the floating cells recovered at the time of medium replacement were cultured on a non-TC culture treated plate (manufactured by Corning Incorporated). On the fifth day from the start of culture in the medium E1, a supernatant containing floating cells was recovered. Next, pipetting and peeling were performed using a release agent ("TrypLE Select" manufactured by Thermo Fisher Scientific) to collect all the cells in the cell culture substrate.

(Examples 2 to 5)

A cell culture substrate was obtained in the same manner as in Example 1, except that the configuration of the cell scaffold in the cell culture substrate was changed as described in the following table. Using the obtained cell culture substrate, "(2) Culture of cells" was performed in the same manner as in Example 1.

(Example 6)

A 24-well plate (manufactured by Corning Incorporated, surface treatment: ultra-low adhesion treatment, bottom area of each well: 1.9 cm$^2$, described as 24wP in the table) was used as a substrate of the cell culture substrate (cell culture substrate body). The configuration of the cell scaffold was changed as described in the following table. A cell culture substrate was obtained in the same manner as in Example 1 except for these. "(2) Culture of cells" was performed in the same manner as in Example 1, except that the obtained cell culture substrate was used, the seeding density of iPS cells on the cell culture substrate was set to $4.0 \times 10^4$ cells/well, and the medium amount was set to 1/2.

(Comparative Example 1)

A cell culture substrate was obtained in the same manner as in Example 1, except that the cell scaffold was disposed on the entire first surface of the substrate (cell culture substrate body). Using the obtained cell culture substrate, "(2) Culture of cells" was performed in the same manner as in Example 1.

(Example 7)

(1) Preparation of cell culture substrate

As a substrate (cell culture substrate body), a 12-well plate (manufactured by Corning Incorporated, surface treatment: tissue culture treatment (TC treatment), bottom area of each well: 3.8 cm$^2$) was prepared. A polyvinyl acetal resin (X) was prepared in the same manner as in Example 1.

Dimethylformamide (DMF) was prepared as a first solvent. Dimethylformamide (DMF) was prepared as a second solvent. As the peptide, a cyclic peptide having an amino acid sequence of Arg-Gly-Asp-Phe-Lys (five amino acid residues, forming a cyclic skeleton by bonding Arg and Lys, Phe is a D form, c-RGDfK) was prepared. As a condensing agent, 1-ethyl-3-(3-dimethylaminopropyl)carbodiimide hydrochloride was prepared. The polyvinyl acetal resin (X) (50 parts by weight) and the peptide (2 parts by weight) were mixed with 1000 parts by weight of the first solvent to prepare a first solution. A condensing agent (1 part by weight) was mixed with 1000 parts by weight of the second solvent to prepare a second solution. The first solution and the second solution were mixed to prepare a solution containing the polyvinyl acetal resin (X), the peptide, and the condensing agent.

The obtained solution was reacted at 40°C for 2 hours, and a carboxyl group in the structural unit derived from acrylic acid of the polyvinyl acetal resin (X) and an amino group of Lys of the peptide were dehydrated and condensed, thereby obtaining a solution containing a peptide-conjugated polyvinyl acetal resin (peptide-conjugated resin).

The obtained solution containing a peptide-conjugated polyvinyl acetal resin was diluted 100 times with DMF, and added dropwise to a column packed with an ion exchange resin (manufactured by Organo Corporation) at a rate of 0.3 mL/min for washing. The solution after washing was vacuum-dried at 60°C for 3 hours to obtain a dried solid, the dried solid was dissolved in butanol (alcohol solvent), and then 5 parts by weight of acetic acid (pH adjusting agent) was added to 100 parts by weight of butanol (alcohol solvent) to obtain a solution containing a peptide-conjugated polyvinyl acetal resin and butanol (alcohol solvent).

The obtained solution containing a peptide-conjugated polyvinyl acetal resin and butanol (alcohol solvent) was applied in a dot pattern on the first surface of the substrate using an inkjet device, and then dried to volatilize the solvent.

In this way, a cell culture substrate including a substrate and a cell scaffold disposed in a dot pattern on the first surface of the substrate was obtained. The cell scaffold contains a peptide-conjugated polyvinyl acetal resin (peptide-conjugated resin).

The details of the obtained cell culture substrate are shown in the following table. Note that the cell scaffold prepared as described above was described as "PVB-AA (composition 2)" in the following table.

(2) Culture of cells

"(2) Culture of cells" was performed in the same manner as in Example 1, except that the obtained cell culture substrate was used and the following points were changed.

(a) As iPS cells, 201B7 line (described as line 2 in the following table) was used.
(b) In place of the media A1 to E1, media A2 to E2 shown in the following table were used.

[Table 6]

| | Basal medium | Added differentiation-inducing factor and concentration | Other components |
|---|---|---|---|
| Medium A2 | Essential-8 medium (Gibco) | VEGF (manufactured by R&D Systems, Inc.) 80 ng/mL<br>BMP4 (manufactured by Miltenyi Biotec) 80 ng/mL<br>CHIR99021 (GSK-3 inhibitor, manufactured by Miltenyi Biotec) 4 μM | 1 × antibiotic-antimycotic solution (manufactured by Sigma-Aldrich Co., Ltd.) |
| Medium B2 | Essential-6 medium (Gibco) | VEGF (manufactured by R&D Systems, Inc.) 80 ng/mL<br>FGF-2 (manufactured by Miltenyi Biotec) 50 ng/mL<br>SCF (manufactured by Miltenyi Biotec) 50 ng/mL<br>S3431542 (ALK5 inhibitor, manufactured by Miltenyi Biotec) 2 μM | 1 × antibiotic-antimycotic solution (manufactured by Sigma-Aldrich Co., Ltd.) |
| Medium C2 | StemPro-34 medium (Gibco) | VEGF (manufactured by R&D Systems, Inc.) 40 ng/mL<br>SCF (manufactured by Miltenyi Biotec) 50 ng/mL<br>TPO (manufactured by Miltenyi Biotec) 10 ng/mL<br>IL-3 (manufactured by Miltenyi Biotec) 50 ng/mL<br>FL (manufactured by Miltenyi Biotec) 50 ng/mL | 1 × antibiotic-antimycotic solution (manufactured by Sigma-Aldrich Co., Ltd.) |
| Medium D2 | StemPro-34 medium (Gibco) | SCF (manufactured by Miltenyi Biotec) 50 ng/mL<br>TPO (manufactured by Miltenyi Biotec) 10 ng/mL<br>IL-3 (manufactured by Miltenyi Biotec) 50 ng/mL<br>FL (manufactured by Miltenyi Biotec) 50 ng/mL<br>M-CSF (manufactured by Miltenyi Biotec) 50 ng/mL | 1 × antibiotic-antimycotic solution (manufactured by Sigma-Aldrich Co., Ltd.) |

(continued)

| | Basal medium | Added differentiation-inducing factor and concentration | Other components |
|---|---|---|---|
| Medium E2 | StemPro-34 medium (Gibco) | FL (manufactured by Miltenyi Biotec) 50 ng/mL<br>M-CSF (manufactured by Miltenyi Biotec) 50 ng/mL<br><br>GM-CSF (manufactured by Miltenyi Biotec) 25 ng/mL | 1 × antibiotic-antimycotic solution (manufactured by Sigma-Aldrich Co., Ltd.) |

(Example 8)

A cell culture substrate was obtained in the same manner as in Example 7, except that the configuration of the cell scaffold in the cell culture substrate was changed as described in the following table. Using the obtained cell culture substrate, "(2) Culture of cells" was performed in the same manner as in Example 7.

(Example 9)

(1) Preparation of cell culture substrate

A mixed liquid of laminin ("iMatrix-511" manufactured by Nippi, Incorporated) and phosphate buffered saline (PBS) was applied in a dot pattern on the first surface of the substrate using a pipetman, and then dried to volatilize the solvent. In this way, a cell culture substrate including a substrate and a cell scaffold disposed in a dot pattern on the first surface of the substrate was obtained. The cell scaffold contains laminin.

(2) Culture of cells

Using the obtained cell culture substrate, "(2) Culture of cells" was performed in the same manner as in Example 7.

(Comparative Example 2)

A cell culture substrate was obtained in the same manner as in Example 7, except that the cell scaffold was disposed on the entire first surface of the substrate (cell culture substrate body). Using the obtained cell culture substrate, "(2) Culture of cells" was performed in the same manner as in Example 7.

(Evaluation: Examples 1 to 9 and Comparative Examples 1 and 2)

(1) Presence of floating cells

On the fifth day (Day 15) from the start of culture in the medium E1 or E2, the cell culture substrate was observed with a microscope to confirm the presence or absence of floating cells. When floating cells are present, it can be estimated that monocytes are produced.

<Evaluation criteria for presence of floating cells>

○: Floating cells can be confirmed
×: Floating cells cannot be confirmed

(2) Number of recovered cells

The number of recovered cells was measured using a cell counter ("NC 3000" manufactured by M&S TechnoSystems, Inc.).

(3) Number of recovered cells per area of 10 mm$^2$ of upper surface of cell scaffold

The number of recovered cells per area of 10 mm$^2$ of the upper surface of the cell scaffold was calculated by dividing the number of recovered cells by the area of the upper surface of the cell scaffold.

(4) Ratio of CD14-positive cells

The recovered cells were labeled using an anti-CD14 antibody (manufactured by BD Pharmingen), and then measured and analyzed using a flow cytometer ("SA3800 cell analyzer" manufactured by Sony Corporation).

The ratio of the CD14-positive cells was calculated by the following formula.

$$\texttt{Ratio (\%) of CD14-positive cells = A/B × 100}$$

A: Number of cells having CD14 marker detected by measurement with flow cytometer (cells)
B: Number of cells measured by flow cytometer (cells)

(5) Induction of differentiation of CD14-positive cells into macrophage-like cells

The CD14-positive cells obtained in Examples 1 to 6 were evaluated as follows. TexMACS medium (manufactured by Miltenyi Biotec) added with 50 ng/mL of M-CSF (granulocyte-macrophage colony-stimulating factor, manufactured by Miltenyi Biotec) was prepared. Into the cell culture substrate, 4 mL of this medium was added, and the CD14-positive cells were cultured for 7 days. During culture, half of the liquid in the cell culture substrate was replaced with fresh medium at a frequency of once every two days. For the cells after culture, induction of differentiation into macrophage-like cells was confirmed by the following method.

(a) The cultured cells were labeled using an anti-CD11b antibody, and then measured using a flow cytometer. As a result, cells expressing CD11b, which is a marker for macrophages, were confirmed in all Examples.
(b) The cultured cells were further cultured for 24 hours in 4 mL of TexMACS medium to which 20 ng/mL of IL-4 was added. Next, the cultured cells were labeled using an anti-CD163 antibody and an anti-CD206 antibody, and then measured using a flow cytometer. As a result, cells expressing CD163 and CD206, which are markers of M2 macrophages, were confirmed in all Examples. When cytokines were confirmed by ELISA, production of IL-10 was confirmed in all Examples.

Details and results are shown in the table below. Note that "Condition 1" in the column of medium conditions in the following table means conditions using the media A1 to E1, and "Condition 2" means conditions using the media A2 to E2. ".E+0N" in the following table means "×10$^N$", and for example, "3.2.E+06" means $3.2×10^6$. Note that the evaluation result of "Induction of differentiation of CD14-positive cells into macrophage-like cells" in Examples 1 to 6 was determined as "o" from the results of (a) and (b) described above.

[Table 7]

| | | | Example 1 | Example 2 | Example 3 | Example 4 | Example 5 | Example 6 | Comparative Example 1 |
|---|---|---|---|---|---|---|---|---|---|
| Cell culture substrate | Substrate | Type | 12wP | 12wP | 12wP | 12wP | 12wP | 24wP | 12wP |
| | | Surface treatment | TC treatment | TC treatment | TC treatment | TC treatment | TC treatment | Ultra-low adhesion treatment | TC treatment |
| | Cell scaffold | Type | PVB-AA (Composition 1) | PVB-AA (Composition 1) | PVB-AA (Composition 1) | PVB-AA (Composition 1) | PVB-AA (Composition 1) | PVB-AA (Composition 1) | PVB-AA (Composition 1) |
| | | Synthetic method | Solid phase method | Solid phase method | Solid phase method | Solid phase method | Solid phase method | Solid phase method | Solid phase method |
| | | Number of cell scaffolds (number of dots) | 106 | 48 | 13 | 13 | 13 | 9 | Whole surface coating |
| | | Upper surface — Average value of aspect ratios | 1 | 1 | 1 | 1 | 1 | 1 | |
| | | Upper surface — Average value ($\mu$m) of equivalent circle diameters | 700 | 700 | 700 | 1500 | 2000 | 1000 | |
| | | Upper surface — Average value (mm) of distances between centers of gravity | 1.3 | 2 | 4 | 4 | 4 | 2 | |
| | | Total area (mm$^2$) of upper surface | 40.8 | 18.5 | 5.0 | 23.0 | 40.8 | 7.1 | 380 |
| iPS cells | | Cell line | Line 1 (053018A) | Line 1 (053018A) | Line 1 (053018A) | Line 1 (053018A) | Line 1 (053018A) | Line 1 (053018A) | Line 1 (053018A) |
| Medium conditions | | | Condition 1 | Condition 1 | Condition 1 | Condition 1 | Condition 1 | Condition 1 | Condition 1 |

(continued)

| | | Example 1 | Example 2 | Example 3 | Example 4 | Example 5 | Example 6 | Comparative Example 1 |
|---|---|---|---|---|---|---|---|---|
| | Presence of floating cells | ○ | ○ | ○ | ○ | ○ | ○ | ○ |
| Differentiation into monocytes | Number of recovered cells (cells/-well) | 3.2.E+06 | 4.9.E+06 | 3.2.E+06 | 2.7.E+06 | 2.9.E+06 | 1.4.E+06 | 3.7.E+05 |
| | Number of recovered cells per area of 10 mm$^2$ of upper surface of cell scaffold (cells/10 mm$^2$) | 7.8.E+05 | 2.7.E+06 | 6.4.E+06 | 1.2.E+06 | 7.1.E+05 | 2.0.E+06 | 9.7.E+03 |
| | Number of CD14-positive cells (cells/well) | 1.6.E+06 | 3.3.E+06 | 2.7.E+06 | 2.1.E+06 | 2.1.E+06 | 1.1.E+06 | 0.0.E+00 |
| | Ratio (%) of CD14-positive cells | 51% | 67% | 84% | 77% | 71% | 80% | 0% |
| Induction of differentiation into macrophage-like cells | | ○ | ○ | ○ | ○ | ○ | ○ | - |

[Table 8]

|  |  |  |  | Example 7 | Example 8 | Example 9 | Comparative Example 2 |
|---|---|---|---|---|---|---|---|
| Cell culture substrate | Substrate | | Type | 12wP | 12wP | 12wP | 12wP |
| | | | Surface treatment | TC treatment | TC treatment | TC treatment | TC treatment |
| | Cell scaffold | | Type | PVB-AA (Composition 2) | PVB-AA (Composition 2) | Laminin | PVB-AA (Composition 2) |
| | | | Synthetic method | Liquid phase method | Liquid phase method | - | Liquid phase method |
| | | | Number of cell scaffolds (number of dots) | 16 | 16 | 16 | Whole surface coating |
| | | Upper surface | Average value of aspect ratios | 1 | 1 | 1 | |
| | | | Average value ($\mu$m) of equivalent circle diameters | 700 | 1000 | 4000 | |
| | | | Average value (mm) of distances between centers of gravity | 4 | 4 | 4 | |
| | | | Total area (mm$^2$) of upper surface | 6.2 | 12.6 | 201.0 | 380 |
| iPS cells | | Cell line | | Line 2 (201B7) | Line 2 (201B7) | Line 2 (201B7) | Line 2 (201B7) |
| Medium conditions | | | | Condition 2 | Condition 2 | Condition 2 | Condition 2 |
| Differentiation into monocytes | | Presence of floating cells | | ○ | ○ | ○ | ○ |
| | | Number of recovered cells (cells/well) | | 3.8.E+06 | 3.4.E+06 | 2.8.E+06 | 4.5.E+05 |
| | | Number of recovered cells per area of 10 mm$^2$ of upper surface of cell scaffold (cells/10 mm$^2$) | | 6.2.E+06 | 2.7.E+06 | 1.4.E+05 | 1.2.E+04 |
| | | Number of CD14-positive cells (cells/well) | | 1.6.E+06 | 1.7.E+06 | 1.1.E+06 | 1.7.E+05 |
| | | Ratio (%) of CD14-positive cells | | 43% | 50% | 39% | 39% |

(Example 10 and 11)

(1) Preparation of cell culture substrate

A cell culture substrate was obtained in the same manner as in Example 7, except that the configuration of the cell scaffold in the cell culture substrate was changed as described in the following table.

(2) Culture of cells

"(2) Culture of cells" was performed in the same manner as in Example 7, except that the obtained cell culture substrate was used and the following points were changed.

(a) As iPS cells, 253G1 (described as line 3 in the following table) was used.
(b) The seeding density of iPS cells on the prepared cell culture substrate was tested under each of four conditions of $2.0\times10^4$ cells/well, $4.0\times10^4$ cells/well, $8.0\times10^4$ cells/well, and $1.6\times10^5$ cells/well.
(c) The steps from "(2-1) Maintenance culture of iPS cells" to "(2-5) Differentiation induction step (step (3), Day 4 to Day 7)" were performed under the same conditions as in Example 7 at the seeding density under the above four conditions. Culture in the medium C2 was then continued for 5 days. During culture in the medium C2, half of the liquid in the cell culture substrate was replaced with a fresh medium C2 at a frequency of once every two days. On Day 9, the supernatant containing suspension cells was collected, and then pipetting and peeling were performed using a release agent ("TrypLE Select" manufactured by Thermo Fisher Scientific) to collect all the cells in the cell culture substrate.

(Comparative Example 3)

A cell culture substrate was obtained in the same manner as in Example 10, except that the cell scaffold was disposed on the entire first surface of the substrate (cell culture substrate body). Using the obtained cell culture substrate, "(2) Culture of cells" was performed in the same manner as in Example 10.

(Example 12)

(1) Preparation of cell culture substrate

A cell culture substrate was obtained in the same manner as in Example 7, except that a 24-well plate (manufactured by Corning Incorporated, surface treatment: tissue culture treatment (TC treatment), bottom area of each well: $1.9$ cm$^2$, described as 24wP in the table) was used as a substrate of the cell culture substrate (cell culture substrate body) and the configuration of the cell scaffold was changed as described in the following table.

(2) Culture of cells

"(2) Culture of cells" was performed in the same manner as in Example 10, except that the obtained cell culture substrate was used.

(Example 13)

(1) Preparation of cell culture substrate

A cell culture substrate was obtained in the same manner as in Example 7, except that a 24-well plate (manufactured by Corning Incorporated, surface treatment: ultra-low adhesion treatment, bottom area of each well: $1.9$ cm$^2$, described as 24wP in the table) was used as a substrate of the cell culture substrate (cell culture substrate body) and the configuration of the cell scaffold was changed as described in the following table.

(2) Culture of cells

Using the obtained cell culture substrate, "(2) Culture of cells" was performed in the same manner as in Example 12.

(Evaluation: Examples 10 to 13 and Comparative Example 3)

(1) Presence of floating cells

On the fifth day from the start of culture in the medium C2, the cell culture substrate was observed with a microscope to confirm the presence or absence of floating cells. When floating cells are present, it can be estimated that hematopoietic progenitor cells are produced.

<Evaluation criteria for presence of floating cells>

**[0224]**

　○: Floating cells can be confirmed
　×: Floating cells cannot be confirmed

(2) Number of recovered cells

The number of recovered cells was measured using a cell counter ("NC 3000" manufactured by M&S TechnoSystems, Inc.).

(3) Number of CD34/CD43-positive cells (cells/well)

The recovered cells were labeled using an anti-CD34 antibody (manufactured by BD Pharmingen) and an anti-CD43 antibody (manufactured by BD Pharmingen), and then measured and analyzed using a flow cytometer ("SA3800 cell analyzer" manufactured by Sony Corporation). The number of cells positive for both CD34 and CD43 (the number of CD34/CD43-positive cells) was calculated.

Details and results are shown in the table below. Note that "Condition 2" in the column of medium conditions in the following table means conditions using the media A2 to E2. "E+0N" in the following table means "×10 to the power of N", and for example, "3.2E+06" means $3.2 \times 10^6$.

[Table 9]

| | | | | Example 10 | Example 11 | Comparative Example 3 |
|---|---|---|---|---|---|---|
| Cell culture substrate | Substrate | Type | | 12wP | 12wP | 12wP |
| | | Surface treatment | | TC treatment | TC treatment | TC treatment |
| | Cell scaffold | Type | | PVB-AA (Composition 2) | PVB-AA (Composition 2) | PVB-AA (Composition 2) |
| | | Synthetic method | | Liquid phase method | Liquid phase method | Liquid phase method |
| | | Number of cell scaffolds (number of dots) | | 16 | 16 | Whole surface coating |
| | | Upper surface | Average value of aspect ratios | 1 | 1 | |
| | | | Average value (μm) of equivalent circle diameters | 700 | 1000 | |
| | | | Average value (mm) of distances between centers of gravity | 4 | 4 | |
| | | Total area (mm²) of upper surface | | 6.2 | 12.6 | 380 |

(continued)

| | | | Example 10 | Example 11 | Comparative Example 3 |
|---|---|---|---|---|---|
| iPS cells | Cell line | | Line 3 (253G1) | Line 3 (253G1) | Line 3 (253G1) |
| | Seeding condition 1 | Seeding density (cells/well) | 2.0E+04 | 2.0E+04 | 2.0E+04 |
| | Seeding condition 2 | Seeding density (cells/well) | 4.0E+04 | 4 .0E+04 | 4.0E+04 |
| | Seeding condition 3 | Seeding density (cells/well) | 8.0E+04 | 8.0E+04 | 8.0E+04 |
| | Seeding condition 4 | Seeding density (cells/well) | 1.6E+05 | 1.6E+05 | 1.6E+05 |
| Medium conditions | | | Condition 2 | Condition 2 | Condition 2 |
| Differentiation into hematopoietic progenitor cells | Seeding condition 1 | Presence of floating cells | ○ | ○ | ○ |
| | | Number of recovered cells (cells/well) | 5.3E+05 | 8.2E+05 | 2.2E+06 |
| | | Number of CD34/CD43-positive cells (cells/well) | 5.1E+03 | 1.1E+04 | 1.4E+04 |
| | Seeding condition 2 | Presence of floating cells | ○ | ○ | ○ |
| | | Number of recovered cells (cells/well) | 1.2E+06 | 1.7E+06 | 2.4E+06 |
| | | Number of CD34/CD43-positive cells (cells/well) | 1.9E+04 | 2.8E+04 | 7.2E+03 |
| | Seeding condition 3 | Presence of floating cells | ○ | ○ | ○ |
| | | Number of recovered cells (cells/well) | 1.9E+06 | 1.5E+06 | 2.4E+06 |
| | | Number of CD34/CD43-positive cells (cells/well) | 5.5E+04 | 1.6E+04 | 8.8E+03 |
| | Seeding condition 4 | Presence of floating cells | ○ | ○ | ○ |
| | | Number of recovered cells (cells/well) | 1.8E+06 | 1.4E+06 | 2.5E+06 |
| | | Number of CD34/CD43-positive cells (cells/well) | 2.9E+04 | 8.9E+03 | 3.2E+03 |

[Table 10]

| | | | | Example 12 | Example 13 |
|---|---|---|---|---|---|
| Cell culture substrate | Substrate | Type | | 24wP | 24wP |
| | | Surface treatment | | TC treatment | Ultra-low adhesion treatment |
| | Cell scaffold | Type | | PVB-AA (Composition 2) | PVB-AA (Composition 2) |
| | | Synthetic method | | Liquid phase method | Liquid phase method |
| | | Number of cell scaffolds (number of dots) | | 9 | 9 |
| | | Upper surface | Average value of aspect ratios | 1 | 1 |
| | | | Average value ($\mu$m) of equivalent circle diameters | 1000 | 1000 |
| | | | Average value (mm) of distances between centers of gravity | 4 | 4 |
| | | Total area (mm$^2$) of upper surface | | 7.1 | 7.1 |
| iPS cells | | Cell line | | Line 3 (253G1) | Line 3 (253G1) |
| | | Seeding condition 1 | Seeding density (cells/well) | 1.0E+04 | 1.0E+04 |
| | | Seeding condition 2 | Seeding density (cells/well) | 2.0E+04 | 2.0E+04 |
| | | Seeding condition 3 | Seeding density (cells/well) | 4.0E+04 | 4.0E+04 |
| | | Seeding condition 4 | Seeding density (cells/well) | 8.0E+04 | 8.0E+04 |
| Medium conditions | | | | Condition 2 | Condition 2 |

(continued)

|  |  |  | Example 12 | Example 13 |
|---|---|---|---|---|
| Differentiation into hematopoietic progenitor cells | Seeding condition 1 | Presence of floating cells | ○ | ○ |
|  |  | Number of recovered cells (cells/-well) | 1.5E+05 | 4.7E+05 |
|  |  | Number of CD34/CD43-positive cells (cells/well) | 1.0E+04 | 2.3E+04 |
|  | Seeding condition 2 | Presence of floating cells | ○ | ○ |
|  |  | Number of recovered cells (cells/-well) | 4.9E+05 | 6.3E+05 |
|  |  | Number of CD34/CD43-positive cells (cells/well) | 2.6E+04 | 4.3E+04 |
|  | Seeding condition 3 | Presence of floating cells | ○ | ○ |
|  |  | Number of recovered cells (cells/-well) | 7.2E+05 | 7.6E+05 |
|  |  | Number of CD34/CD43-positive cells (cells/well) | 4.6E+04 | 5.9E+04 |
|  | Seeding condition 4 | Presence of floating cells | ○ | ○ |
|  |  | Number of recovered cells (cells/-well) | 9.8E+05 | 7.8E+05 |
|  |  | Number of CD34/CD43-positive cells (cells/well) | 3.2E+04 | 5.5E+04 |

As shown in Table 9, it can be seen that in Examples 10 and 11, hematopoietic progenitor cells can be stably produced even at a high seeding density, as compared with Comparative Example 3. As shown in Table 10, it can be seen that more hematopoietic progenitor cells are obtained in Example 13 using the substrate subjected to an ultra-low adhesion treatment than in Example 12 under any seeding condition.

(Example 14)

(1) Preparation of cell culture substrate

A cell culture substrate was obtained in the same manner as in Example 7, except that the configuration of the cell scaffold was changed as described in the following table.

(2) Culture of cells

The steps from "(2-1) Maintenance culture of iPS cells" to "(2-5) Differentiation induction step (step (3), Day 4 to Day 7)" were performed in the same manner as in Example 7, except that the obtained cell culture substrate was used and StemFit AK02N was used instead of StemFit AK03N.
The following media F1 and G1 for differentiation induction were prepared.
Medium F1: StemSpan Lymphoid Progenitor Expansion Medium (STEMCELL Technologies)

Medium G1: StemSpan NK Cell Differentiation Medium (STEMCELL Technologies)

(2-6) Differentiation induction step (step (4B), Day 7 to Day 21)

The medium C2 in the cell culture substrate was removed, 1.6 mL of the medium F1 was added into the cell culture substrate, and the cells were cultured for 14 days under the conditions of 37°C and a $CO_2$ concentration of 5% in a state in which the cell population containing hematopoietic progenitor cells adhered onto the surface of the cell scaffold. During culture, half of the liquid in the cell culture substrate was replaced with a fresh medium F1 at a frequency of once every three days. In this way, a cell population containing lymphoid progenitor cells was obtained.

(2-7) Differentiation induction step (step (5B), Day 21 to Day 44)

The medium F1 in the cell culture substrate was removed, 1.6 mL of the medium G1 was added into the cell culture substrate, and the cells were cultured under the conditions of 37°C and a $CO_2$ concentration of 5% in a state in which the cell population containing lymphoid progenitor cells adhered onto the surface of the cell scaffold. During culture, half of the liquid in the cell culture substrate was replaced with a fresh medium G1 at a frequency of once every three days. Note that the floating cells recovered at the time of medium replacement were cultured on a non-TC culture treated plate (manufactured by Corning Incorporated). On Day 44, a supernatant containing floating cells and cells detached by pipetting were collected. Next, pipetting and peeling were performed using a release agent ("TrypLE Select" manufactured by Thermo Fisher Scientific) to collect all the cells in the cell culture substrate. In this way, a cell population containing lymphocytes (NK cells) was obtained.

(Example 15)

A cell culture substrate was obtained in the same manner as in Example 14, except that the configuration of the cell scaffold in the cell culture substrate was changed as described in the following table. Using the obtained cell culture substrate, "(2) Culture of cells" was performed in the same manner as in Example 14.

(Comparative Example 4)

A cell culture substrate was obtained in the same manner as in Example 7, except that the cell scaffold was disposed on the entire first surface of the substrate (cell culture substrate body). Using the obtained cell culture substrate, "(2) Culture of cells" was performed in the same manner as in Example 14.

(Evaluation: Examples 14 and 15 and Comparative Example 4)

(1) Presence of floating cells

On the 23rd day (Day 44) from the start of culture in the medium G1, the cell culture substrate was observed with a microscope to confirm the presence or absence of floating cells. When floating cells are present, it can be estimated that lymphocytes (NK cells) are produced.

<Evaluation criteria for presence of floating cells>

○: Floating cells can be confirmed
×: Floating cells cannot be confirmed

(2) Ratio of cells detached by pipetting

Before using a release agent, the ratio of cells detached from the cell culture substrate by pipetting (the number of cells detached by pipetting/the number of cells adhering to the surface of the cell scaffold before pipetting ×100 (%)) was visually confirmed.

(3) Number of iPS cells at Day 0

The number of iPS cells at Day 0 (cells/well) was measured using a cell counter ("NC3000" manufactured by M&S TechnoSystems, Inc.).

(4) Number of CD56-positive cells

The recovered cells were labeled using an anti-CD56 antibody (manufactured by BD Pharmingen), and then measured and analyzed using a flow cytometer ("SA3800 cell analyzer" manufactured by Sony Corporation).
The ratio of the CD56-positive cells was calculated by the following formula, and the number of CD56-positive cells (cells/well) was calculated from the recovered number of cells at Day 44.

$$\text{Ratio (\%) of CD56-positive cells} = A/B \times 100$$

A: Number of cells having CD56 marker detected by measurement with flow cytometer (cells)
B: Number of cells measured by flow cytometer (cells)

Details and results are shown in the table below. Note that "Condition 3" in the column of medium conditions in the following table means conditions using the media A2 to C2, F1, and G1. ".E+0N" in the following table means "$\times 10$ to the power of N", and for example, "3.2.E+06" means $3.2 \times 10^6$.

[Table 11]

| | | | | Example 14 | Example 15 | Comparative Example 4 |
|---|---|---|---|---|---|---|
| Cell culture substrate | Substrate | Type | | 12wP | 12wP | 12wP |
| | | Surface treatment | | TC treatment | TC treatment | TC treatment |
| | Cell scaffold | Type | | PVB-AA (Composition 2) | PVB-AA (Composition 2) | PVB-AA (Composition 2) |
| | | Synthetic method | | Liquid phase method | Liquid phase method | Liquid phase method |
| | | Number of cell scaffolds (number of dots) | | 16 | 16 | Whole surface coating |
| | | Upper surface | Average value of aspect ratios | 1 | 1 | |
| | | | Average value ($\mu$m) of equivalent circle diameters | 500 | 700 | |
| | | | Average value (mm) of distances between centers of gravity | 4 | 4 | |
| iPS cells | | Cell line | | Line 2 (201B7) | Line 2 (201B7) | Line 2 (201B7) |
| Medium conditions | | | | Condition 3 | Condition 3 | Condition 3 |
| Differentiation into NK cells | | Presence of floating cells | | ○ | ○ | ○ |
| | | Ratio of cells detached by pipetting | | About 50% | About 50% | About 20% |
| | | Number of iPS cells at Day 0 (cells/well) | | 6.8.E+04 | 6.6.E+04 | 1.2.E+06 |
| | | Number of CD56-positive cells (cells/well) | | 1.3.E+05 | 7.5.E+04 | 7.4.E+05 |
| | | Number of CD56-positive cells/-number of iPS cells at Day 0 | | 1.96 | 1.14 | 0.64 |
| | | Number of CD56-positive cells per area of 1 cm$^2$ of upper surface of cell scaffold (cells/cm$^2$) | | 4.2.E+06 | 1.2.E+06 | 3.9.E+05 |

As shown in Table 11, in Examples 14 and 15, as compared with Comparative Example 4, a larger number of CD56-positive cells were obtained per number of iPS cells at Day 0 and per area of 1 cm$^2$ of the upper surface of the cell scaffold.

(Example 16)

(1) Preparation of cell culture substrate

A cell culture substrate A1 was obtained in the same manner as in Example 7, except that the configuration of the cell scaffold was changed as described in the following table.

A solution prepared by diluting a rhDLL4/Fc chimeric protein solution (10 μg/mL, manufactured by Sino Biological) with an equal amount of a retronectin solution (10 μg/mL, manufactured by Takara Bio Inc.) was added in an amount of 1 mL to each well of a 6-well plate (surface treatment: tissue culture treatment (TC treatment)), and the plate was incubated at 4°C overnight. In this way, a cell culture substrate B1 was obtained. Similarly, a solution prepared by diluting a rhDLL4/Fc chimeric protein solution (10 μg/mL, manufactured by Sino Biological) with an equal amount of a retronectin solution (10 μg/mL, manufactured by Takara Bio Inc.) was added in an amount of 0.4 mL to each well of a 24-well plate (surface treatment: tissue culture treatment (TC treatment)), and the plate was incubated at 4°C overnight. In this way, a cell culture substrate B2 was obtained.

(2) Culture of cells

Media A3 to C3 and F2 for differentiation induction shown in the following table were prepared.

[Table 12]

|  | Basal medium | Added differentiation-inducing factor and concentration | Other components |
|---|---|---|---|
| Medium A3 | StemPro-34 medium (Gibco) | BMP4 (manufactured by Miltenyi Biotec) 50 ng/mL<br>VEGF (manufactured by R&D Systems, Inc.) 50 ng/mL<br>bFGF (manufactured by Fujifilm Corporation) 50 ng/mL<br>CHIR99021 (GSK-3 inhibitor, manufactured by Miltenyi Biotec) 4 μM | Glutamax (manufactured by Thermo Fisher Scientific) 2 mM<br>Ascorbic acid-2-phosphate 50 μg/mL<br><br>Monothioglycerol 0.4 mM 1×ITS-G (manufactured by Thermo Fisher Scientific) |
| Medium B3 | StemPro-34 medium (Gibco) | VEGF (manufactured by R&D Systems, Inc.) 50 ng/mL<br>bFGF (manufactured by Fujifilm Corporation) 50 ng/mL<br><br>SCF (manufactured by Miltenyi Biotec) 50 ng/mL | Glutamax (manufactured by Thermo Fisher Scientific) 2 mM<br>Ascorbic acid 2-phosphate 50 μg/mL<br>Monothioglycerol 0.4 mM 1×ITS-G (manufactured by Thermo Fisher Scientific) |
| Medium C3 | StemPro-34 medium (Gibco) | VEGF (manufactured by R&D Systems, Inc.) 50 ng/mL<br>bFGF (manufactured by Fujifilm Corporation) 50 ng/mL<br>SCF (manufactured by Miltenyi Biotec) 50 ng/mL<br>TPO (manufactured by Miltenyi Biotec) 30 ng/mL<br>FLT-3L (manufactured by Miltenyi Biotec) 10 ng/mL | Glutamax (manufactured by Thermo Fisher Scientific) 2 mM<br><br>Ascorbic acid 2-phosphate 50 μg/mL<br>Monothioglycerol 0.4 mM 1×ITS-G (manufactured by Thermo Fisher Scientific) |
| Medium F2 | StemSpan SFEM II medium (STEMCELL Technologies) | SCF (manufactured by Miltenyi Biotec) 50 ng/mL<br>TPO (manufactured by Miltenyi Biotec) 50 ng/mL<br>IL-7 (manufactured by Miltenyi Biotec) 50 ng/mL<br>FLT-3L (manufactured by Miltenyi Biotec) 50 ng/mL<br>SDF-1α (manufactured by Miltenyi Biotec) 30 nM<br>SB203580 (p38MAPK inhibitor, manufactured by Tocris Bioscience) 15 μM | Glutamax (manufactured by Thermo Fisher Scientific) 2 mM<br><br>Ascorbic acid 2-phosphate 50 μg/mL<br><br>2-ME 50 μM |

The steps from " (2-1) Maintenance culture of iPS cells" to "(2-2) Culture of iPS cells (cell cluster forming step)" were performed in the same manner as in Example 10, except that the obtained cell culture substrate A1 was used, and 6 wells of the cell culture substrate A1 were seeded at a seeding density of $1.6 \times 10^5$ cells/well.

(2-3) Differentiation induction step (step (1), Day 0 to Day 4)

Into the cell culture substrate A1, 1 mL of the medium A3 was added, and the cells were cultured for 4 days under the conditions of 37°C and a $CO_2$ concentration of 5% in a state in which the cell cluster adhered onto the surface of the cell scaffold. On the second day (Day 2) from the start of culture in the medium A3, 6 $\mu$M of SB431542 (manufactured by FUJIFILM Wako Pure Chemical Corporation) was added. In this way, a cell population including mesoderm was obtained.

(2-4) Differentiation induction step (step (2), Day 4 to Day 7)

The medium A3 in the cell culture substrate A1 was removed, 1 mL of the medium B3 was added into the cell culture substrate A1, and the cells were cultured for 3 days under the conditions of 37°C and a $CO_2$ concentration of 5% in a state in which the cell population containing mesoderm adhered onto the surface of the cell scaffold. In this way, a cell population including hemogenic endothelial cells was obtained.

(2-5) Differentiation induction step (step (3), Day 7 to Day 15)

The medium B3 in the cell culture substrate A1 was removed, 1 mL of the medium C3 was added into the cell culture substrate A1, and the cells were cultured for 8 days under the conditions of 37°C and a $CO_2$ concentration of 5% in a state in which the cell population containing hemogenic endothelial cells adhered onto the surface of the cell scaffold. During culture, the entire amount of the liquid in the cell culture substrate A1 was replaced with a fresh medium C3 at a frequency of once every two days until Day 11, and the medium was not replaced on and after Day 11. In this way, a cell population containing hematopoietic progenitor cells was obtained.

(2-6) Differentiation induction step (steps (4B) to (5B), Day 15 to Day 37)

The cells in the cell culture substrate A1 were detached using a release agent ("TrypLE Select" manufactured by Thermo Fisher Scientific) under the conditions of 37°C and 15 minutes and recovered. The recovered cells were taken in and out about 15 times with 2.5 ML 21G (manufactured by Terumo Corporation) with a syringe injection needle to be finely divided, and then only single cells were collected with a 40 $\mu$m cell strainer (manufactured by BD Japan Ltd.). CD34-positive cells were recovered from the collected cells by the magnet bead method of Easy Sep Human CD34 Positive Selection Kit II (Stemcell Technologies). The number of recovered CD34-positive cells was measured using a cell counter ("TC20" manufactured by BioRad).
CD34-positive cells of $2.0 \times 10^4$ cells among the recovered CD34-positive cells were seeded on the cell culture substrate B1, 2 mL of the medium F2 was added into the cell culture substrate B1, and the cells were cultured for 8 days at 37°C and a $CO_2$ concentration of 5%. On Day 17 and Day 23, the liquid in the cell culture substrate B1 was recovered into a tube and centrifuged, the medium F2 was removed and replaced with the same amount of a fresh medium F2, and returned to the original cell culture substrate B1. On Day 30, the liquid in the cell culture substrate B1 was recovered into a tube and centrifuged, the medium F2 was removed and replaced with 400 $\mu$L of a fresh medium F2, and then seeded on the newly prepared cell culture substrate B2. On Day 36, the liquid in the cell culture substrate B2 was recovered into a tube and centrifuged, the medium F2 was removed and replaced with the same amount of a fresh medium F2. On Day 37, all cells in the cell culture substrate B2 were recovered by pipetting.

(Example 17)

(1) Preparation of cell culture substrate

A cell culture substrate A2 was obtained in the same manner as in Example 7, except that the configuration of the cell scaffold was changed as described in the following table.
A solution prepared by diluting a rhDLL4/Fc chimeric protein solution (10 $\mu$g/mL, manufactured by Sino Biological) with an equal amount of a retronectin solution (10 $\mu$g/mL, manufactured by Takara Bio Inc.) was added in an amount of 0.4 mL to each well of a 48-well plate (surface treatment: tissue culture treatment (TC treatment)), and the plate was incubated at 4°C overnight. In this way, a cell culture substrate B3 was obtained. Similarly, a solution prepared by diluting a rhDLL4/Fc chimeric protein solution (10 $\mu$g/mL, manufactured by Sino Biological) with an equal amount of a retronectin solution (10 ug/mL, manufactured by Takara Bio Inc.) was added in an amount of 0.8 mL to each well of a 12-well plate (surface treatment: tissue culture treatment (TC treatment)), and the plate was incubated at 4°C overnight. In this way, a cell culture substrate B4 was obtained.

(2) Culture of cells

Media A4 to C4 and F3 for differentiation induction shown in the following table were prepared.

[Table 13]

| | Basal medium | Added differentiation-inducing factor and concentration | Other components |
|---|---|---|---|
| Medium A4 | StemPro-34 medium (Gibco) | VEGF (manufactured by R&D Systems, Inc.) 80 ng/mL<br>BMP4 (manufactured by Miltenyi Biotec) 80 ng/mL<br>FGF-2 (manufactured by Miltenyi Biotec) 50 ng/mL<br>CHIR99021 (GSK-3 inhibitor, manufactured by Miltenyi Biotec) 4 $\mu$M | Glutamax (manufactured by Thermo Fisher Scientific) 2 mM<br>Ascorbic acid-2-phosphate 50 $\mu$g/mL<br>Monothioglycerol 0.4 mM<br>1$\times$ITS-G (manufactured by Thermo Fisher Scientific) |
| Medium B4 | StemPro-34 medium (Gibco) | VEGF (manufactured by R&D Systems, Inc.) 80 ng/mL<br>FGF-2 (manufactured by Miltenyi Biotec) 50 ng/mL<br>SCF (manufactured by Miltenyi Biotec) 50 ng/mL<br>SB431542 (ALK5 inhibitor, manufactured by Miltenyi Biotec) 2 $\mu$M | Glutamax (manufactured by Thermo Fisher Scientific) 2 mM<br>Ascorbic acid 2-phosphate 50 $\mu$g/mL<br>Monothioglycerol 0.4 mM<br>1$\times$ITS-G (manufactured by Thermo Fisher Scientific) |
| Medium C4 | StemPro-34 medium (Gibco) | VEGF (manufactured by R&D Systems, Inc.) 40 ng/mL<br>SCF (manufactured by Miltenyi Biotec) 50 ng/mL<br>TPO (manufactured by Miltenyi Biotec) 10 ng/mL<br>IL-3 (manufactured by Miltenyi Biotec) 50 ng/mL<br>FLT-3L (manufactured by Miltenyi Biotec) 50 ng/mL | Glutamax (manufactured by Thermo Fisher Scientific) 2 mM<br>Ascorbic acid 2-phosphate 50 $\mu$g/mL<br>Monothioglycerol 0.4 mM<br>1$\times$ITS-G (manufactured by Thermo Fisher Scientific) |
| Medium F3 | StemSpan SFEM II medium (STEMCELL Technologies) | SCF (manufactured by Miltenyi Biotec) 50 ng/mL<br>TPO (manufactured by Miltenyi Biotec) 50 ng/mL<br>IL-7 (manufactured by Miltenyi Biotec) 50 ng/mL<br>FLT-3L (manufactured by Miltenyi Biotec) 50 ng/mL<br>SDF-1$\alpha$ (manufactured by Miltenyi Biotec) 30 nM<br>SB203580 (p38MAPK inhibitor, manufactured by Tocris Bioscience) 15 $\mu$M | Glutamax (manufactured by Thermo Fisher Scientific) 2 mM<br>Ascorbic acid 2-phosphate 50 $\mu$g/mL<br>2-ME 55 $\mu$M |

The steps from "(2-1) Maintenance culture of iPS cells" to "(2-2) Culture of iPS cells (cell cluster forming step)" were performed in the same manner as in Example 10, except that the obtained cell culture substrate A2 was used, and 1 well of the cell culture substrate A2 was seeded at a seeding density of $1.4\times10^5$ cells/well.

(2-3) Differentiation induction step (step (1), Day 0 to Day 2)

Into the cell culture substrate A2, 1 mL of the medium A4 was added, and the cells were cultured for 2 days under the conditions of 37°C and a $CO_2$ concentration of 5% in a state in which the cell cluster adhered onto the surface of the cell scaffold. In this way, a cell population including mesoderm was obtained.

(2-4) Differentiation induction step (step (2), Day 2 to Day 4)

The medium A4 in the cell culture substrate A2 was removed, 1 mL of the medium B4 was added into the cell culture substrate A2, and the cells were cultured for 2 days under the conditions of 37°C and a $CO_2$ concentration of 5% in a state in which the cell population containing mesoderm adhered onto the surface of the cell scaffold. In this way, a cell population including hemogenic endothelial cells was obtained.

(2-5) Differentiation induction step (step (3), Day 4 to Day 14)

The medium B4 in the cell culture substrate A2 was removed, 1 mL of the medium C4 was added into the cell culture substrate A2, and the cells were cultured for 10 days under the conditions of 37°C and a $CO_2$ concentration of 5% in a state in which the cell population containing hemogenic endothelial cells adhered onto the surface of the cell scaffold. During culture, the entire amount of the liquid in the cell culture substrate A2 was replaced with a fresh medium C4 at a frequency of once every two days until Day 11, and the medium was not replaced on and after Day 11. In this way, a cell population containing hematopoietic progenitor cells was obtained.

(2-6) Differentiation induction step (steps (4B) to (5B), Day 14 to Day 33)

Cells floating in the cell culture substrate A2 (cells floating without adhering to the cell scaffold) were collected using a pipette. CD34-positive cells were recovered from the recovered cells by the magnet bead method of Easy Sep Human CD34 Positive Selection Kit II (Stemcell Technologies). The number of recovered CD34-positive cells was measured using a cell counter ("TC20" manufactured by BioRad).

CD34-positive cells of $1.28 \times 10^4$ cells among the recovered CD34-positive cells were seeded on the cell culture substrate B3, 0.5 mL of the medium F3 was added into the cell culture substrate B3, and the cells were cultured for 14 days at 37°C and a $CO_2$ concentration of 5%. On Day 19, Day 21, Day 23, and Day 26, the liquid in the cell culture substrate B3 was recovered into a tube and centrifuged, the medium F3 was removed and replaced with the same amount of a fresh medium F3, and returned to the original cell culture substrate B3. On Day 28, the liquid in the cell culture substrate B3 was recovered into a tube and centrifuged, the medium F3 was removed and replaced with 1 mL of a fresh medium F3, and then seeded on the newly prepared cell culture substrate B4. On Day 33, all cells in the cell culture substrate B4 were recovered by pipetting.

(Example 18)

The steps up to Day 13 of "(2-5) Differentiation induction step (step (3)" were performed in the same manner as in Example 17.

(2-6) Differentiation induction step (steps (4B) to (5B), Day 13 to Day 34)

Cells floating in the cell culture substrate A2 (cells floating without adhering to the cell scaffold) were collected using a pipette. The number of recovered cells was measured using a cell counter ("TC20" manufactured by BioRad). Some of the recovered cells were sampled and labeled using an anti-CD34 antibody (manufactured by BD Pharmingen), and then measured and analyzed using a flow cytometer ("BD FACSLyric" manufactured by BD) to determine the CD34 positive rate. The number of the recovered cells was multiplied by the CD34 positive rate to determine the number of the recovered CD34-positive cells.

Cells recovered from the cell culture substrate A2 were seeded on the cell culture substrate B4 so that the number of CD34-positive cells was $3.6 \times 10^2$ cells, 1 mL of the medium F3 was added to the cell culture substrate B4, and the cells were cultured for 21 days at 37°C and a $CO_2$ concentration of 5%. On Day 18, the liquid in the cell culture substrate B4 was recovered into a tube and centrifuged, the medium F3 was removed and replaced with the same amount of a fresh medium F3, and returned to the original cell culture substrate B4. On Day 20 and Day 22, the liquid in the cell culture substrate B4 was recovered into a tube and centrifuged, the medium F3 was removed and replaced with 1 mL of a fresh medium F3, and then seeded on the newly prepared cell culture substrate B4. On Day 25, Day 28, and Day 32, the liquid in the cell culture substrate B4 was recovered into a tube and centrifuged, the medium F3 was removed and replaced with the same amount of a fresh medium F3, and returned to the original cell culture substrate B4. On Day 35, all cells in the cell culture substrate B4 were recovered by pipetting.

(Evaluation: Examples 16 to 18)

(1) Number of recovered cells

The number of recovered cells was measured using a cell counter ("TC20" manufactured by BioRad).

(2) Number of CD3-positive cells

The recovered cells were labeled using a FITC-anti-CD3 antibody (manufactured by BD Biosciences), and then measured and analyzed using a flow cytometer ("Lyric" manufactured by BD Japan Ltd.) to calculate the number of CD3-positive cells.

(3) Number of CD3-positive cells/number of iPS cells

The number of CD3-positive cells obtained from one iPS cell (the number of CD3-positive cells/the number of iPS cells) was calculated by the following formula.

(Number of CD3-positive cells/number of iPS cells) = A/B × C/D

A: Number of CD3-positive cells (cells)

B: Number of seeded CD34-positive cells (cells)

C: Number of recovered CD34-positive cells (cells)

D: Number of seeded iPS cells (cells)

Details and results are shown in the table below. Note that ".E+0N" in the following table means "$\times 10^N$", and for example, "3.2.E+06" means $3.2\times10^6$.

[Table 14]

| | | | | Example 16 |
|---|---|---|---|---|
| Cell culture substrate A1 | Substrate | Type | | 12wP |
| | | Surface treatment | | TC treatment |
| | Cell scaffold | Type | | PVB-AA (Composition 2) |
| | | Synthetic method | | Liquid phase method |
| | | Number of cell scaffolds (number of dots) | | 16 |
| | | Upper surface | Average value of aspect ratios | 1 |
| | | | Average value ($\mu$m) of equivalent circle diameters | 500 |
| | | | Average value (mm) of distances between centers of gravity | 4 |
| Cell culture substrate B | Substrate | Type (cell culture substrate B1) | | 6wP |
| | | Type (cell culture substrate B2) | | 24wP |
| | | Surface treatment | | TC treatment |
| | Cell scaffold | Type | | DLL/Retronectin |
| | | Coating method | | Whole surface coating |
| iPS cells | | Cell line | | Line 3 (253G1) |
| | | Number of seeded iPS cells (cells) | | 9.6.E+05 |
| Hematopoietic progenitor cells | | Number of recovered CD34-positive cells (cells) | | 4.4.E+04 |
| | | Number of seeded CD34-positive cells (cells) | | 2.0.E+04 |

(continued)

|  |  | Example 16 |
|---|---|---|
| Differentiation into T cells | Number of recovered cells (cells) | 2.3.E+05 |
|  | Number of CD3-positive cells (cells) | 1.0.E+05 |
|  | Number of CD3-positive cells/number of iPS cells | 0.2 |

[Table 15]

| | | | Example 17 | Example 18 |
|---|---|---|---|---|
| Cell culture substrate A2 | Substrate | Type | 12wP | 12wP |
| | | Surface treatment | TC treatment | TC treatment |
| | Cell scaffold | Type | PVB-AA (Composition 2) | PVB-AA (Composition 2) |
| | | Synthetic method | Liquid phase method | Liquid phase method |
| | | Number of cell scaffolds (number of dots) | 16 | 16 |
| | | Upper surface — Average value of aspect ratios | 1 | 1 |
| | | Upper surface — Average value (μm) of equivalent circle diameters | 700 | 700 |
| | | Upper surface — Average value (mm) of distances between centers of gravity | 4 | 4 |
| Cell culture substrate B | Substrate | Type (cell culture substrate B3) | 48wP | - |
| | | Type (cell culture substrate B4) | 12wP | 12wP |
| | | Surface treatment | TC treatment | TC treatment |
| | Cell scaffold | Type | DLL/Retronectin | DLL/Retronectin |
| | | Coating method | Whole surface coating | Whole surface coating |
| iPS cells | | Cell line | Line 3 (253G1) | Line 3 (253G1) |
| | | Number of seeded iPS cells (cells) | 1.4.E+05 | 1.4.E+05 |
| Hematopoietic progenitor cells | | Number of recovered CD34-positive cells (cells) | 7.7.E+04 | 6.7.E+04 |
| | | Number of seeded CD34-positive cells (cells) | 1.3.E+04 | 3.6.E+02 |
| Differentiation into T cells | | Number of recovered cells (cells) | 4.6.E+05 | 5.4.E+05 |
| | | Number of CD3-positive cells (cells) | 5.6.E+04 | 2.9.E+04 |
| | | Number of CD3-positive cells/number of iPS cells | 2.5 | 39.7 |

As shown in Tables 14 and 15, it is found that CD3-positive cells were obtained in Examples 16 to 18. It is found that in Examples 17 and 18 including the step of recovering floating cells in the obtained cell population containing hematopoietic progenitor cells and the step of culturing the recovered cells in the presence of a medium containing a differentiation-inducing factor to obtain a cell population containing lymphoid progenitor cells, the number of CD3-positive cells obtained from one iPS cell increased as compared with Example 16.

EXPLANATION OF SYMBOLS

1: Cell culture substrate
2: Substrate
2a: First surface
3: Cell scaffold
3a: Upper surface
D: Diameter (equivalent circle diameter)
H: Height
L: Distance between centers of gravity

**Claims**

1.  A method for producing hematopoietic progenitor cells, the method comprising:

    a cell cluster forming step of culturing pluripotent stem cells using a cell culture substrate including a substrate and a cell scaffold disposed in a dot pattern on a first surface of the substrate and forming a cell cluster on a surface of the cell scaffold; and
    a differentiation induction step of inducing differentiation of the obtained cell cluster into a cell population containing hematopoietic progenitor cells.

2.  The method for producing hematopoietic progenitor cells according to claim 1, wherein an average value of equivalent circle diameters of an upper surface of the cell scaffold is 200 $\mu$m or more and 3000 $\mu$m or less.

3.  The method for producing hematopoietic progenitor cells according to claim 1 or 2, wherein an average value of distances between centers of gravity of an upper surface of the cell scaffold is 0.5 mm or more and 10 mm or less.

4.  The method for producing hematopoietic progenitor cells according to any one of claims 1 to 3, wherein the cell scaffold contains a peptide-conjugated resin having a synthetic resin moiety and a peptide moiety.

5.  The method for producing hematopoietic progenitor cells according to claim 4, wherein the peptide-conjugated resin has a polyvinyl acetal resin moiety and a peptide moiety.

6.  The method for producing hematopoietic progenitor cells according to any one of claims 1 to 5, wherein a region where the cell scaffold is not disposed on the first surface of the substrate has a region subjected to a cell low adhesion treatment.

7.  The method for producing hematopoietic progenitor cells according to any one of claims 1 to 6, wherein the pluripotent stem cells are iPS cells.

8.  A method for producing leukocytes, the method comprising a step of inducing differentiation of a cell population containing hematopoietic progenitor cells obtained by the method for producing hematopoietic progenitor cells according to any one of claims 1 to 7 into a cell population containing monocytes, lymphocytes, or granulocytes.

9.  A method for producing leukocytes, the method comprising:

    a cell cluster forming step of culturing pluripotent stem cells using a cell culture substrate including a substrate and a cell scaffold disposed in a dot pattern on a first surface of the substrate and forming a cell cluster on a surface of the cell scaffold; and
    a differentiation induction step of inducing differentiation of the obtained cell cluster into a cell population containing monocytes, lymphocytes, or granulocytes.

10. The method for producing leukocytes according to claim 9, wherein an average value of equivalent circle diameters of an upper surface of the cell scaffold is 200 $\mu$m or more and 3000 $\mu$m or less.

11. The method for producing leukocytes according to claim 9 or 10, wherein an average value of distances between centers of gravity of an upper surface of the cell scaffold is 0.5 mm or more and 10 mm or less.

12. The method for producing leukocytes according to any one of claims 9 to 11, wherein the cell scaffold contains a peptide-conjugated resin having a synthetic resin moiety and a peptide moiety.

13. The method for producing leukocytes according to claim 12, wherein the peptide-conjugated resin has a polyvinyl acetal resin moiety and a peptide moiety.

14. The method for producing leukocytes according to any one of claims 9 to 13, wherein a region where the cell scaffold is not disposed on the first surface of the substrate has a region subjected to a cell low adhesion treatment.

15. The method for producing leukocytes according to any one of claims 9 to 14, wherein the pluripotent stem cells are iPS cells.

[FIG. 1]

(a)

(b)

[FIG. 2]

## INTERNATIONAL SEARCH REPORT

| International application No. |
|---|
| **PCT/JP2024/019905** |

| A. | CLASSIFICATION OF SUBJECT MATTER |
|---|---|

*C12N 5/07*(2010.01)i; *C12N 5/02*(2006.01)i; *C12N 5/10*(2006.01)i; *C12N 5/078*(2010.01)i; *C12N 5/0786*(2010.01)i; *C12N 5/0787*(2010.01)i; *C12N 5/0789*(2010.01)i

FI: C12N5/07; C12N5/0786; C12N5/0787; C12N5/078; C12N5/02; C12N5/10 ZNA; C12N5/0789

According to International Patent Classification (IPC) or to both national classification and IPC

| B. | FIELDS SEARCHED |
|---|---|

Minimum documentation searched (classification system followed by classification symbols)

C12N5/07; C12N5/02; C12N5/10; C12N5/078; C12N5/0786; C12N5/0787; C12N5/0789

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2021-185810 A (DAI NIPPON PRINTING CO., LTD.) 13 December 2021 (2021-12-13) claims 13-14, paragraphs [0044], [0077], [0089], [0131] | 1-3, 6-11, 14-15 |
| Y | | 1-15 |
| Y | WO 2021/024943 A1 (SEKISUI CHEMICAL CO., LTD.) 11 February 2021 (2021-02-11) claim 1, paragraphs [0107], [0132] | 1-15 |
| Y | WO 2021/106832 A1 (KYOTO UNIVERSITY) 03 June 2021 (2021-06-03) paragraphs [0031], [0055] | 1-15 |
| Y | US 2016/0186137 A1 (WISCONSIN ALUMNI RESEARCH FOUNDATION) 30 June 2016 (2016-06-30) claim 1 | 1-15 |
| A | JP 2021-158960 A (TOSOH CORP.) 11 October 2021 (2021-10-11) | 1-15 |
| A | WO 2021/090594 A1 (THE UNIVERSITY OF TOKYO) 14 May 2021 (2021-05-14) | 1-15 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 July 2024** | **30 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/019905**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.

**PCT/JP2024/019905**

| Patent document cited in search report | | | Publication date (day/month/year) | Patent family member(s) | | | Publication date (day/month/year) |
|---|---|---|---|---|---|---|---|
| JP | 2021-185810 | A | 13 December 2021 | (Family: none) | | | |
| WO | 2021/024943 | A1 | 11 February 2021 | US | 2022/0235307 | A1 | |
| | | | | claim 1, paragraphs [0119], [0143] | | | |
| | | | | EP | 4008770 | A1 | |
| | | | | CN | 113924356 | A | |
| WO | 2021/106832 | A1 | 03 June 2021 | US | 2023/0000915 | A1 | |
| | | | | paragraphs [0079], [0104]-[0105] | | | |
| | | | | EP | 4067490 | A1 | |
| | | | | CN | 114761560 | A | |
| | | | | KR | 10-2022-0106975 | A | |
| US | 2016/0186137 | A1 | 30 June 2016 | (Family: none) | | | |
| JP | 2021-158960 | A | 11 October 2021 | (Family: none) | | | |
| WO | 2021/090594 | A1 | 14 May 2021 | (Family: none) | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021158960 A

- WO 2021024943 A1

**Non-patent literature cited in the description**

- *The Journal of Cell Biology*, September 1995, vol. 130 (5), 1189-1196
- *PROTEIN, NUCLEIC ACID, AND ENZYME*, 2000, vol. 45 (15), 2477

- *Pathophysiology*, 1990, vol. 9 (7), 527-535
- *Journal of Osaka Women's and Children's Hospital*, 1992, vol. 8 (1), 58-66